(19) **Europäisches Patentamt**
**European Patent Office**
**Office européen des brevets**

(11) **EP 4 644 905 A1**

(12) **EUROPEAN PATENT APPLICATION**
published in accordance with Art. 153(4) EPC

(43) Date of publication:
**05.11.2025 Bulletin 2025/45**

(21) Application number: **23912301.1**

(22) Date of filing: **28.12.2023**

(51) International Patent Classification (IPC):
***G01N 33/86*** (2006.01)

(52) Cooperative Patent Classification (CPC):
**G01N 33/86**

(86) International application number:
**PCT/JP2023/047187**

(87) International publication number:
**WO 2024/143524 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.12.2022 JP 2022212338**

(71) Applicants:
• **Sekisui Medical Co., Ltd.**
**Tokyo 1030027 (JP)**
• **Public University Corporation
Nara Medical University
Nara 6348521 (JP)**

(72) Inventors:
• **KAWABE, Toshiki**
**Tokyo 103-0027 (JP)**
• **ONISHI, Kengo**
**Tokyo 103-0027 (JP)**
• **NOGAMI, Keiji**
**Kashihara-shi, Nara 6348521 (JP)**
• **OGIWARA, Kenichi**
**Kashihara-shi, Nara 6348521 (JP)**
• **SHIMONISHI, Naruto**
**Kashihara-shi, Nara 6348521 (JP)**

(74) Representative: **Wächtershäuser & Hartz
Patentanwaltspartnerschaft mbB
Weinstraße 8
80333 München (DE)**

(54) **METHOD FOR ANALYZING BLOOD CLOTTING ABILITY OF BLOOD SAMPLE**

(57) A method for analyzing a blood coagulation ability of a blood specimen. The method includes: 1) acquiring a first derivative $V(i)$ of a coagulation reaction curve of a subject blood specimen, where $i$ denotes the number of measurement points or time; and 2) the following a) or b): a) determining $p_k$, which is a minimum of a point at which $V(i)$ reaches $X_k$ or higher when $V(i)$ reaches a maximum $Vmax$ or therebefore, and $q_k$, which is a maximum of a point at which $V(i)$ reaches $X_k$ or higher when $V(i)$ reaches the maximum $Vmax$ or thereafter, where $k$ represents a series of integers from 1 to $n$, $n$ is an integer greater than or equal to two, $X_k$ is $Vmax \times S_k$ %, and $0 < S_k \leq 100$; b) determining a relativized maximum of $V(i)$.

**Description**

Technical Field

The present invention relates to a method for analyzing a blood coagulation ability of a blood specimen.

Background Art

Hemophilia A is a hemorrhagic disease caused by deficiency or dysfunction of coagulation factor VIII (FVIII). Hemophilia A is classified into congenital hemophilia A with congenital deficiency of FVIII, and acquired hemophilia A caused by autoantibody (inhibitor) to FVIII. Some patients with congenital hemophilia A have inhibitors. Hemophilia A is classified into mild, moderate, and severe depending on the FVIII activity level in the blood. Specimens derived from patients with hemophilia A have a prolonged blood coagulation time in a blood coagulation test depending on the severity.

Emicizumab, which is a hemophilia A treatment, is a recombinant humanized bispecific antibody which can promote the blood coagulation reaction. Emicizumab can bind activated coagulation factor IX (FIXa) at one antigen-binding site, bind coagulation factor X (FX) at the other antigen-binding site, and substitute for the cofactor function of activated coagulation factor VIII (FVIIIa), thereby allowing the blood coagulation reaction to be promoted.

Emicizumab is regularly administered to patients with hemophilia A for the purpose of suppressing the bleeding tendency of patients with hemophilia A. The administered emicizumab remains in the blood for a certain period, during which a prolonged blood coagulation time, i.e., a typical characteristic of coagulation abnormality, is not observed in a blood coagulation test of a blood specimen obtained by drawing blood from a patient with hemophilia A to whom emicizumab has been administered. Accordingly, the result shows as if there were no coagulation abnormality. Meanwhile, there is a possibility that reduction in prolonged coagulation time of a patient with hemophilia A (to the reference range upper limit or less) by emicizumab poses a significant danger to the patient. For example, in a case where a patient with hemophilia A undergoes emergency transport and a surgical operation due to a traffic accident or the like, and no prolonged coagulation time (activated partial thromboplastin time: APTT) is observed in a typical preoperative blood coagulation test, a doctor who does not know that the patient has hemophilia A cannot find the patient's reduction in coagulation ability and cannot administer an appropriate treatment. Accordingly, a risk of serious intraoperative bleeding occurs.

Patent Literature 1 describes a method for evaluating a blood coagulation reaction with a substance having a FVIII-substituting activity, by adding a blood coagulation initiation reagent containing activated coagulation factor XI to a blood specimen, and measuring the amount of generated thrombin. Patent Literatures 2 and 3 describe a method for evaluating a coagulation ability of a blood specimen, the method including: acquiring a parameter about the derivative of a coagulation waveform about the blood specimen to which a substance having a FVIII-substituting activity has been administered; acquiring a FVIII activity as a value indicating the coagulation ability from the parameter; and evaluating the coagulation ability of the blood specimen, based on the acquired FVIII activity.

Citation List

Patent Literature

Patent Literature 1: WO 2014/050926
Patent Literature 2: WO 2016/170944
Patent Literature 3: JP-A-2017-106925

Summary of Invention

Technical Problem

The present invention provides a method for analyzing a blood coagulation ability of a blood specimen, the method allowing detecting the blood specimen to which a drug having FVIII-substituting activity has been administered.

Solution to Problem

The present invention provides the following.

[1] A method for analyzing a blood coagulation ability of a blood specimen, the method comprising:

1) acquiring a first derivative V(i) of a coagulation reaction curve of a subject blood specimen in which presence or absence of a substance having coagulation factor VIII-substituting activity is required to be detected, where i denotes the number of measurement points or time; and
2) the following a) or b):

a) determining $p_k$, which is a minimum of a point at which V(i) reaches $X_k$ or higher when V(i) reaches a maximum Vmax or therebefore, and $q_k$, which is a maximum of a point at which V(i) reaches $X_k$ or higher when V(i) reaches the maximum Vmax or thereafter, where k represents a series of integers from 1 to n, n is an integer greater than or equal to two, $X_k$ is Vmax $\times$ $S_k$%, and $0 < S_k \leq 100$;
b) determining a relativized maximum of V(i).

[2] The method according to [1], wherein the subject blood specimen is a blood specimen with no prolonged activated partial thromboplastin time (APTT).

[3] The method according to [1] or [2], wherein the n is an integer from 5 to 50.

[4] The method according to any one of [1] to [3], further comprising calculating a parameter for analyzing the blood coagulation ability of the subject blood specimen, based on the $p_k$ or $q_k$.

[5] The method according to [4],

wherein the parameter is at least one selected from the group consisting of a pre-post average difference, CV, qp ratio, $vB(S_k)$, kurtosis $vAB(S_k)$, rate over time $vTB(S_k)$, $vAB(S_k) \times$ VmaxT, $vAB(S_k) \times$ APTT, parameters about relative values of $p_k$ and $q_k$, and their standard deviation indices (SDI), where
the pre-post average difference represents (average of $q_a$ to $q_b$ - average of $p_a$ to $p_b$)/(average of $p_a$ to $p_b$ and $q_a$ to $q_b$) (where a and b are integers selected from the group consisting of 1 to n, n is as defined above, and a < b),
the CV represents a coefficient of variation of $p_a$ to $p_b$ and $q_a$ to $q_b$ (where a and b are integers selected from the group consisting of 1 to n, n is as defined above, and a < b),
the qp ratio represents $q_k/p_k$,
the $vB(S_k)$ represents an interval from $p_k$ to $q_k$,
the VmaxT satisfies V(VmaxT) = Vmax,
the APTT is an activated partial thromboplastin time (APTT) of the subject blood specimen,
the $vAB(S_k) = vH(S_k)/vB(S_k)$, and
the $vTB(S_k) = vB(S_k)/VmaxT$,
where
the $vH(S_k)$ is of the following equation,

$$vH(Sk) = \frac{\sum_{i=pk}^{qk}\left(i \times V(i)\right)}{\sum_{i=pk}^{qk} i}$$

[6] The method according to [5],

wherein the parameters about the relative values of the $p_k$ and $q_k$ are slopes of linear regression lines of a plot from $p_a$ to $p_b$ of the subject blood specimen and a plot from $q_c$ to $q_d$ of the subject blood specimen with respect to $p_a$ to $p_b$ of a reference specimen and $q_c$ to $q_d$ of a reference specimen, respectively (where a, b, c, and d are integers each independently selected from the group consisting of 1 to n, a < b, c < d, and n is as defined above), and
the reference specimen is a blood specimen to which no drug having FVIII-substituting activity has been administered.

[7] The method according to any one of [1] to [3], further comprising calculating a parameter for analyzing the blood coagulation ability of the subject blood specimen, based on the relativized maximum of V(i).

[8] The method according to [7],

wherein the parameter is at least one selected from the group consisting of a corrected Vmax, the corrected Vmax $\times$ the VmaxT, the corrected Vmax $\times$ the APTT, and their standard deviation indices (SDI),
the corrected Vmax is the relativized maximum of V(i),

the VmaxT satisfies V(VmaxT) = Vmax, and

the APTT is an activated partial thromboplastin time (APTT) of the subject blood specimen.

[9] The method according to any one of [4] to [8], further comprising detecting the presence or absence of a substance having coagulation factor VIII-substituting activity in the subject blood specimen, based on the parameter.

[10] The method according to [9], further comprising outputting a result of the detecting the presence or absence of a substance having coagulation factor VIII-substituting activity.

[11] The method according to any one of [1] to [10], further comprising:

measuring the activated partial thromboplastin time (APTT) of a blood specimen; and

selecting the blood specimen with no prolonged APTT, as the subject blood specimen in which the presence or absence of a substance having coagulation factor VIII-substituting activity is required to be detected.

[12] The method according to any one of [1] to [11], wherein the substance having coagulation factor VIII-substituting activity is a bispecific antibody substituting for a cofactor function of coagulation factor VIII.

[13] The method according to any one of [1] to [12], wherein the substance having coagulation factor VIII-substituting activity is emicizumab.

[14] A program for implementing the method according to any one of [1] to [13].

[15] A device for implementing the method according to any one of [1] to [13].

[16] A system for implementing the method according to any one of [1] to [13], the system comprising: a program for implementing the method according to any one of [1] to [13]; and a device controlled by the program.

[17] The device according to [15] or the system according to [16], further comprising an output unit for outputting a result of analysis of the blood coagulation ability of the blood specimen by the method according to any one of [1] to [131].

Advantageous Effect of Invention

The present invention can detect that a test specimen is a blood specimen to which a drug having FVIII-substituting activity, such as emicizumab, has been administered, among blood specimens in which no coagulation abnormality can be detected by conventional APTT measurement, using measured data (coagulation reaction data) of a coagulation reaction acquired during APTT measurement.

Brief Description of Drawings

Fig. 1 illustrates an example of a coagulation reaction curve.

Fig. 2 is a conceptual diagram for describing $X_k$, $p_k$, and $q_k$.

Fig. 3A illustrates an example of a blood coagulation analyzing process by a method of the present invention.

Fig. 3B is a conceptual diagram illustrating a configuration of a device for performing a method for analyzing a blood coagulation ability by the present invention.

Fig. 4 illustrates APTT of a specimen group used in Example. NE: control specimen group, E1: specimen derived from a patient with hemophilia A to whom emicizumab has been administered. E2: specimen which contains an inhibitor and has been derived from a patient with hemophilia A to whom emicizumab has been administered.

Fig. 5 illustrates coagulation reaction curves R(i) (upper) and first derivative curves V(i) (lower) of NE, E1, and E2.

Fig. 6 illustrates corrected reaction rate curves of NE, E1, and E2.

Fig. 7 illustrates A: a plot of Vmax against APTT of each specimen, and B: distributions of Vmax of each specimen group.

Fig. 8 illustrates A to D: plots of Vmax against APTT of NE, E1, E2, and all the specimens, and E: a distribution of VmaxT of each specimen group.

Fig. 9 illustrates A: a plot of corrected Vmax against APTT of each specimen, where dotted line indicates a threshold line, and B: a distribution of corrected Vmax of each specimen group.

Fig. 10 illustrates A: a plot of [corrected Vmax × VmaxT] against APTT of each specimen, and B: a distribution of [corrected Vmax × VmaxT] of each specimen group.

Fig. 11 illustrates A: a plot of [corrected Vmax × APTT] against APTT of each specimen, and B: a distribution of [corrected Vmax × APTT] of each specimen group.

Fig. 12 illustrates A: a plot of vTB(30) (= vB(30)/VmaxT) against APTT of each specimen, and B: a distribution of vTB(30) of each specimen group.

Fig. 13 illustrates A: a plot of vAB(10) (= vH(10)/vB(10)) against APTT of each specimen, where dotted line indicates a threshold line, and B: a distribution of vAB(10) of each specimen group.

Fig. 14 illustrates A: a plot of [vAB(10) $\times$ VmaxT] against APTT of each specimen, B: a distribution of [vAB(10) $\times$ VmaxT] of each specimen group, C: a plot of [vAB(10) $\times$ APTT] against APTT of each specimen, and D: a distribution of [vAB(10) $\times$ APTT] of each specimen group.

Fig. 15 illustrates A: a plot of a qp ratio($q_8/p_8$) to APTT of each specimen, and B: a distribution of the pq ratio of each specimen group.

Fig. 16 illustrates A: a plot of $q_6 - p_6$ to APTT of each specimen, where dotted line indicates a threshold line, and B: a distribution of $q_6 - p_6$ of each specimen group.

Fig. 17 illustrates A: a plot of a pre-post average difference against APTT of each specimen, B: a distribution of the pre-post average difference of each specimen group, C: a plot of a lower-side pre-post average difference against APTT of each specimen, D: a distribution of the lower-side pre-post average difference of each specimen group, E: a plot of a middle-side pre-post average difference against APTT of each specimen, and F: a distribution of the middle-side pre-post average difference of each specimen group.

Fig. 18 illustrates A: a plot of all CV against APTT of each specimen, B: a distribution of all CV of each specimen group, C: a plot of lower CV against APTT, and D: a distribution of lower CV of each specimen group.

Fig. 19 illustrates a slope of a regression line of relative $p_k$ and relative $q_k$ (k = 1 to 20), A and B: in a case where an NE specimen with minimum APTT is employed as a reference specimen ($\bullet$), a plot of the slope of the regression line against APTT (A), and a distribution of each specimen group (B), and C and D: in a case where the NE specimen with maximum APTT is employed as the reference specimen($\bullet$), a plot of the slope of the regression line against APTT (C), and a distribution of each specimen group (D).

Fig. 20 illustrates the slope of the regression line of the relative $p_k$ and the relative $q_k$ (k = 6 to 20), A and B: in a case where the NE specimen with minimum APTT is employed as the reference specimen ($\bullet$), a plot of the slope of the regression line against APTT (A), and a distribution of each specimen group (B), and C and D: in a case where the NE specimen with maximum APTT is employed as the reference specimen($\bullet$), a plot of the slope of the regression line against APTT (C), and a distribution of each specimen group (D).

Description of Embodiments

All Patent Literatures, Non Patent Literatures, and other publications cited in the present Description are incorporated herein by reference in their entirety.

In a blood coagulation test for measuring the activated partial thromboplastin time (APTT), a predetermined reagent is added to a subject blood specimen, a subsequent blood coagulation reaction is measured, and the blood coagulation time (APTT) is calculated from the coagulation reaction data. To measure the blood coagulation reaction, general means is used; for example, optical means for measuring the scattered light intensity, transmittance, and absorbance, or mechanical means for measuring the viscosity of plasma are used. Typically, the blood coagulation reaction is represented by a coagulation reaction curve which indicates the temporal change in the amount of coagulation reaction. In the following Description, the blood specimen, blood coagulation reaction, and blood coagulation time are sometimes simply called the specimen, coagulation reaction, and coagulation time, respectively.

With specimens having a coagulation abnormality factor, APTT is often prolonged in comparison with that of a normal specimen with no coagulation abnormality. The prolongation of APTT is one of major indicators for the coagulation abnormality related to a bleeding risk or a thrombosis risk. With a specimen derived from a patient with hemophilia A, APTT is typically prolonged beyond a reference range (a range allowing a specimen having APTT within this range to be regarded "normal"). Meanwhile, in a case where a substance having FVIII-substituting activity (hereinafter, also called "FVIII-substituting substance"), such as emicizumab, has been administered to a patient with hemophilia A, APTT of a specimen of the patient is not prolonged, and falls within the reference range or is reduced below the reference range. It is difficult to distinguish by APTT measurement whether the test specimen is a specimen derived from a patient with hemophilia A to which the FVIII-substituting substance has been administered or not based on the reference range of APTT.

The present inventors found that even in a case where no prolonged coagulation time is observed by measuring APTT, the blood specimen derived from a patient with hemophilia A to whom emicizumab has been administered exhibits a different progression of the blood coagulation reaction (blood coagulation reaction rate curve) from that of a specimen to which no emicizumab has been administered.

In the present invention, the presence or absence of FVIII-substituting substance in the test specimen is detected based on coagulation reaction data of a test specimen measured during APTT measurement, specifically, a parameter derived from the blood coagulation rate curve obtained by the first derivative of the coagulation reaction curve. In the present invention, among specimens in which no coagulation abnormality is detected by conventional APTT measurement, a specimen to which the FVIII-substituting substance has been administered can be detected.

In the present Description, the FVIII-substituting substance refers to a substance that can substitute for an FVIII cofactor function. The FVIII cofactor function refers to a function of activated FVIII (FVIIIa) forming a complex of activated coagulation factor IX (FIXa) and coagulation factor X (FX). An example of the FVIII-substituting substance may be a bispecific antibody substituting for the FVIII cofactor function (for example, a bispecific antibody simulating the FVIII structure). An example of such a bispecific antibody may be emicizumab. Emicizumab is a recombinant humanized bispecific antibody that binds the activated coagulation factor IX (FIXa) at one antigen-binding site, and binds the coagulation factor X (FX) at the other antigen-binding site, and substitutes for the FVIII cofactor function. Emicizumab is a drug for suppressing the bleeding tendency of congenital and acquired hemophilia A. Furthermore, next-generation FIXa/FX bispecific antibodies (Mim8, NXT007 and the like) having an action mechanism similar to that of emicizumab have also been developed.

[Method for analyzing blood coagulation ability of blood specimen]

The method for analyzing a blood coagulation ability of a blood specimen in the present invention (hereinafter also called the method of the present invention) can detect the difference between the coagulation abilities of blood specimens which cannot be discriminated based on the coagulation time (APTT), as the difference in progression of the coagulation reaction (coagulation reaction rate curve). In the method of the present invention, based on the first derivative of the coagulation reaction curve of the test specimen, a parameter for analyzing the blood coagulation ability of the test specimen is acquired.

The method of the present invention is described below.

1. Coagulation reaction measurement

Preferably, the method of the present invention uses, as the test specimen, the plasma of a subject drawn for a coagulation test. An anticoagulant typically used for a coagulation test may be added to the specimen. For example, blood is drawn using a blood collection tube which contains sodium citrate, and is subsequently centrifugally separated, thus obtaining plasma.

The coagulation reaction curve of a test specimen used for the method of the present invention is calculated from coagulation reaction data acquired in accordance with procedures for measuring the coagulation reaction in typical APTT measurement. Specifically, in coagulation reaction measurement, an APTT measurement reagent is added to the test specimen, and the blood coagulation reaction is started. The coagulation reaction in a mixture containing the reagent and the test specimen is measured. APTT measurement reagents are commercially available (for example, APTT reagent Coagpia APTT-N, and Coagpia APTT-N calcium chloride liquid, which are manufactured by Sekisui Medical Co., Ltd.). To measure the coagulation reaction, general means is used; for example, optical means for measuring the scattered light intensity, transmittance, absorbance or the like, or mechanical means for measuring the viscosity of blood are used. In the following present Description, the method of the present invention is described using, as an example, coagulation reaction measurement based on the scattered light intensity.

The reaction initiation time point of the coagulation reaction can typically be defined as a time point at which the reagent is mixed into the specimen and the coagulation reaction is started. Alternatively, other timing may be defined as a reaction initiation time point. The duration of measuring the coagulation reaction can range, for example, from several tens of seconds to about seven minutes from the time point of mixing the specimen and the reagent. The measurement time may be a freely defined fixed value, or may be up to a time point at which the end of the coagulation reaction of each specimen is detected. During the measurement time, measurement of the progress of the coagulation reaction (measurement of the scattered light intensity) can be repeatedly performed at predetermined intervals. For example, measurement may be performed at intervals of 0.1 seconds. The temperature of the mixture during measurement is under a typical condition, for example, between 30°C and 40°C, inclusive, preferably, between 35°C and 39°C, inclusive. Various conditions of measurement can be set in conformity with the test specimen, reagent, measurement means and the like as appropriate.

A series of operations in the coagulation reaction measurement described above can be performed using an automatic analysis device. An example of the automatic analysis device may be a blood coagulation automatic analysis device CP3000 (manufactured by Sekisui Medical Co., Ltd). Alternatively, some operations may be manually performed. For example, preparation of the test specimen can be performed by a person, and operations thereafter can be performed by the automatic analysis device.

2. Acquisition of coagulation reaction curve and first derivative V(i)

A coagulation reaction curve R(i) is acquired from the measured coagulation reaction data. Here, "i" denotes the number of measurement points or time from the coagulation reaction initiation (also simply called the number of measurement points, and time, respectively). For example, in the case where a measurement (photometry) interval is 0.1 seconds, it is represented that time = 0.1 × the number of measurement points. That is, R(i) may be a function of the number of measurement points, or a function of time. Typically, the coagulation reaction curve R(i) is obtained by applying a denoising or smoothing process to measurements of the coagulation reaction measurement by typical means, or applying zero adjustment for adjusting the initial value or curve relativizing as required.

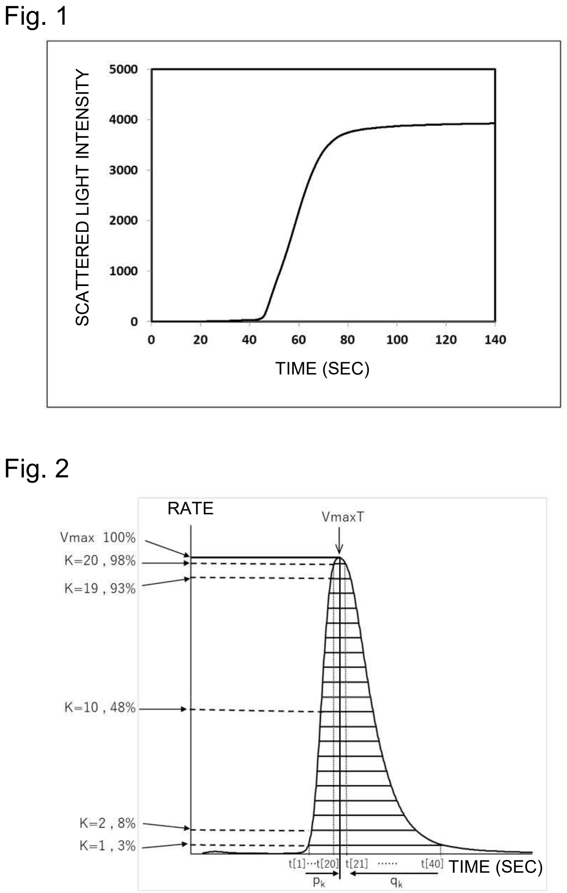

Figure 1 illustrates an example of the coagulation reaction curve. The abscissa axis of Figure 1 indicates time, and the ordinate axis indicates the coagulation reaction (scattered light intensity), which are adjusted such that the scattered light intensity at the measurement initiation time point is zero. The coagulation reaction of the mixture proceeds over time. Thus, the scattered light intensity increases. The coagulation reaction curve based on the scattered light intensity as illustrated in Figure 1 is typically sigmoidal.

From the R(i), its first derivative V(i) is acquired. The differential process for obtaining V(i) from R(i) can be executed by any method, and can be performed by, for example, calculating the intra-interval average slope. In the intra-interval average slope calculation, a predetermined number of measurement points before and after each measurement point i, for example, 2K + 1 measurement points from i - K to i + K can be used. Here, K is any integer. For example, in a case where K is two, (i-2)-, (i-1)-, i-, (i+1)-, and (i+2)-th five measurement points can be used. The average slope means a slope when these measurement points are linearly approximated. A usual method, such as a least-squares method, can be used as the computation method for the linear approximation. The average slope of these measurement points can be assumed as the first derivative V(i) of R(i) at the measurement point i. Alternatively, the first derivative of the relative value of R(i) may be acquired as V(i). For example, conversion may be performed such that the maximum of R(i) is a predetermined value, for example, 100, and the first derivative may be obtained, thus acquiring V(i). The first derivative V(i) constitutes a curve representing the rate of the coagulation reaction, or the reaction progress rate.

The acquisition of R(i) and V(i) about the test specimen may be performed in parallel with the measurement of the coagulation reaction of the test specimen, or performed after the measurement of the coagulation reaction is finished. Preferably, the measurement of the coagulation reaction of the test specimen is performed until the coagulation reaction is finished. The end of the coagulation reaction can be determined based on any reference, such as a time point at which R(i) reaches a plateau, after V(i) reaches the maximum, a time point at which it reduces to 0 or a certain value (for example, S% of the maximum or less). Alternatively, as long as the measurements necessary for obtaining an after-mentioned coagulation time (APTT) and a parameter for analyzing the blood coagulation ability of the test specimen are obtained, the coagulation reaction measurement may be ended at a timing before the coagulation reaction is finished.

As described above, each of R(i) and V(i) in the present Description may be a function of the number of measurement points, or a function of time. The after-mentioned parameter for analyzing the blood coagulation ability of the test specimen may be data with reference to the number of measurement points, or data with reference to time. In the following present Description, R(i) and V(i) are sometimes simply abbreviated as R and V, respectively.

3. APTT calculation

The coagulation time (APTT) of the test specimen can be calculated from the coagulation reaction data acquired by the coagulation reaction measurement. The method of calculating APTT is not specifically limited. For example, APTT can be calculated by any method based on the R(i) or V(i). Examples of the APTT calculating method include: a method of calculating, as the coagulation time, a time point at which R(i) reaches N% of R(E) representing the reaction R at the coagulation reaction end point E (percent detection method); a method of calculating, as the coagulation time, a time point at which V(i) reaches the maximum Vmax or N% thereof; a method of calculating the coagulation time based on the temporal change in the accumulated ratio of R(i) in a minute time period (see JP-A-H6-249855, and WO 2021/132552); a method of calculating the coagulation time based on the weighted average time of V(i) (see WO 2021/177452); and a method of calculating, as the coagulation time, a time point after V(i) reaches the maximum Vmax and then R(i) reaches N% of R(Te) where the timing reaching the predetermined value is assumed as the calculation starting point Te (see WO 2021/206107).

4. Acquisition of parameter for analyzing blood coagulation ability

4.1) Test specimen in which parameter should be acquired

Next, in the method of the present invention, the parameter for analyzing the blood coagulation ability of the test specimen is acquired based on the V(i). In the method of the present invention, the parameter may be acquired for the test specimen in which the presence or absence of the FVIII-substituting substance is required to be detected. Typically, the test specimen in which the presence or absence of the FVIII-substituting substance is required to be detected is a specimen with no prolonged APTT. Without the need to check for the presence or absence of the FVIII-substituting substance, the specimen with prolonged APTT is estimated as a specimen with a coagulation abnormality (that is, a high bleeding risk or thrombosis risk). Accordingly, application of the method of the present invention is not necessarily required.

In one embodiment of the method of the present invention, APTT is calculated based on the coagulation reaction data obtained by measuring any test specimen, and subsequently, a specimen with no prolonged APTT is selected as a test specimen in which the presence or absence of the FVIII-substituting substance is required to be detected. About the selected test specimen, $V(i)$ calculated from the coagulation reaction data obtained by measuring the coagulation reaction can be acquired. The $V(i)$ may be calculated together with the $R(i)$ and the like during APTT measurement, or $V(i)$ may be obtained by measuring APTT, selecting a test specimen with no prolonged APTT to which the method of the present invention should be applied, and then performing calculation based on $R(i)$ of the selected test specimen. In another embodiment of the method of the present invention, from existing coagulation reaction data, a specimen which has been already identified to have no prolonged APTT is selected as a test specimen in which the presence or absence of the FVIII-substituting substance is required to be detected. From the existing coagulation reaction data, $V(i)$ can be acquired.

4.2) Parameter based on peak shape of V

In one embodiment, the parameter for analyzing the blood coagulation ability of the test specimen is a parameter in which the progression of the coagulation reaction is reflected, that is, a parameter based on the peak shape of V. In the acquisition of the parameter, first, a minimum $p_k$ and a maximum $q_k$ of the number of measurement points or time at which V reaches a preset setting value $X_k$ or greater (that is, satisfies $V(i) \geq X_k$) are determined. Typically, V has a peak shape having the maximum Vmax as a peak top. Accordingly, $p_k$ and $q_k$ can be present at a time point when V reaches Vmax or therebefore or thereafter. That is, $V(p_k - D) < X_k \leq V(p_k)$, and $V(q_k) \geq X_k > V(q_k + D)$. Here, D denotes a measurement interval for coagulation reaction data, $D = 1$ in a case where $p_k$ and $q_k$ are with reference to the number of measurement points, and $D = 0.1$ in a case where $p_k$ and $q_k$ are with reference to time, for example, measurement is performed at intervals of 0.1 seconds. In the method of the present invention, $X_k$ denotes a variable, and $p_k$ and $q_k$ corresponding to $X_k$ are determined. For example, in a case where n $X_k$ are present as $(X_1, ..., X_n)$ (k denotes a series of integers from 1 to n, and n denotes an integer greater than or equal to two), a point $p_k$ at which $V(i)$ reaches Vmax or reaches $X_k$ therebefore and a point $q_k$ at which $V(i)$ reaches Vmax or reaches $X_k$ thereafter are determined, and n $p_k$ $(p_1, ..., p_n)$ and n $q_k$ $(q_1, ..., q_n)$ are obtained. As described above, $p_k$ and $q_k$ may be data with reference to the number of measurement points, or data with reference to time. If the coagulation reaction data includes large noise, a peak of V caused by the noise appears, and $p_k$ and $q_k$ are possibly erroneously detected. Erroneous detection of $p_k$ and $q_k$ can be prevented by removing noise in the coagulation reaction data before acquisition of V, or by removing a peak of V due to the noise or $p_k$ and $q_k$ derived therefrom.

The value of the variable $X_k$ may be freely set. Preferably, $X_k$ is greater than 0 and less than or equal to Vmax. Preferably, $X_k$ can be set with reference to Vmax. For example, $X_k$ is defined as Vmax $\times S_k$ (%), where $S_k$ can be greater than 0 and less than or equal to 100, preferably, set in a range from 0.5 to 99. Preferably, $S_k = a + (k - 1) \times b$, where $a > 0$, $b > 0$, and k is as defined above. When $a = 1$ and $b = 1$, $S_k = k$. The maximum of $S_k$ is 100. Note that when $S_k$ is 100, $X_k = $ Vmax, and $p_k = q_k$. For example, in a case where n $X_k$ $(X_1, ..., X_n)$ are set, n $S_k$ $(S_1, ..., S_n)$ are set (here, k and n are as described above, and each $S_k$ is greater than 0 and less than or equal to 100, preferably, from 0.5 to 99, and more preferably, from 5 to 50). The number of set variables $X_k$ is not specifically limited. Preferably, the number ranges from 5 to 50, and more preferably, from 10 to 30, that is, n is an integer ranging, preferably from 5 to 50, and more preferably, from 10 to 30. Preferably, n $S_k$ are made up of a series of values gradually changing from their minimum to maximum at regular intervals.

That is, $p_k$ and $q_k$ satisfy the following formulae.

$$V(p_k - D) < X_k \leq V(p_k), \text{ and } V(q_k) \geq X_k > V(q_k + D),$$

where D denotes the measurement interval.

Note that

$p_k \leq VmaxT \leq q_k$, where VmaxT is the number of measurement points or time point which satisfies $V(VmaxT) = $ Vmax.

$$X_k = Vmax \times S_k \; (\%),$$

$0 < S_k \leq 100$, preferably, $0.5 \leq S_k \leq 99$, and more preferably, $5 \leq S_k \leq 50$.

k denotes an integer ranging from 1 to n,

n denotes an integer ranging, preferably, from 5 to 50, and more preferably, from 10 to 30.

Referring to Figure 2, $X_k$, $p_k$, and $q_k$ are described. In Figure 2, the first derivative V of the coagulation reaction curve is plotted against time. The peak top of V is the maximum Vmax (100%), and a time point at which $V = $ Vmax is represented as VmaxT. 20 $X_k$ (that is, k is an integer ranging from 1 to 20) are set, and are each defined as a value gradually increasing from Vmax $\times$ 3% (k = 1) to Vmax $\times$ 98% (k = 20) at regular intervals (5% increments). Lines each indicating $X_k$

are drawn below the curve of V. For each $X_k$, there are two time points at which V is $X_k$ (intersections between the line of $X_k$ and V). One is present before VmaxT, and the other is present after VmaxT. As a result, for 20 $X_k$, 40 time points at which V = $X_k$ is satisfied are detected. Among these time points, 20 items (from t[1] to t[20]) before VmaxT correspond to $p_k$, and 20 items (from t[40] to t[21]) after VmaxT correspond to $q_k$.

Based on the $p_k$ and $q_k$ described above, the parameter for analyzing the blood coagulation ability of the test specimen can be calculated. An example of the parameter may be the difference of averages of $p_k$ and $q_k$ (hereinafter also called the pre-post average difference). In the present Description, the average of point $p_k$ present before V reaches Vmax is sometimes called pre-Ave. Likewise, the average of point $q_k$ present after V reaches Vmax is sometimes called post-Ave, and the average of all $p_k$ and $q_k$ is sometimes called all-Ave. For example, pre-Ave, post-Ave, and all-Ave can be calculated from all $p_k$ ($p_1$, ..., $p_n$) and all $q_k$ ($q_1$, ..., $q_n$) (n is as defined above). Alternatively, pre-Ave, post-Ave, and all-Ave can be calculated as averages of $p_k$ and $q_k$ in any range (for example, ($p_a$, ..., $p_b$) and ($q_a$, ..., $q_b$), where a and b are integers independently selected from the group consisting of 1 to n, a < b, and n is as defined above). In one embodiment, the pre-post average difference is relativize, and is defined as, for example, the ratio of the difference between pre-Ave and post-Ave to all-Ave ([(post-Ave - pre-Ave)/all-Ave]).

Another example of the parameter may be the coefficient of variation of $p_k$ and $q_k$ (hereinafter, also called CV). For example, CV can be defined as the coefficient of variation (%) of all $p_k$ ($p_1$, ..., $p_n$) and all $q_k$ ($q_1$, ..., $q_n$) (n is as defined above). Alternatively, CV can be defined as the coefficient of variation (%) of $p_k$ and $q_k$ in any range (for example, ($pa$, ..., $p_b$) and ($q_a$, ..., $q_b$). where a and b are integers independently selected from the group consisting of 1 to n, a < b, and n is as defined above). As described in after-mentioned Example, the size of the peak width of V of the specimen containing the FVIII-substituting substance, or the asymmetric degree of the peak shape is reflected in the pre-post average difference and CV.

Another example of the parameter may be the ratio of $q_k$ and $p_k$ (that is, $q_k/p_k$; hereinafter, also called the qp ratio) (k is any integer selected from the group consisting of 1 to n, and n is as defined above). Another example of the parameter may be a reference length $vB(S_k)$, which represents an interval from $p_k$ to $q_k$ (time length or the number of measurement points) (k denotes an integer selected from the group consisting of 1 to n, and n is as defined above). In a case where V is an unimodal peak shape as illustrated in Figure 2, $vB(S_k)$ corresponds to an interval from $p_k$ to $q_k$ (= [$q_k$ - $p_k$], or = [$q_k$ - $p_k$ + D], D is defined as described above). As described in after-mentioned Example, the size of the peak width of V of the specimen containing the FVIII-substituting substance, or the asymmetric degree of the peak shape is reflected also in the qp ratio and $vB(S_k)$.

Another example of the parameter may be parameters about the relative values of $p_k$ and $q_k$. Hereinafter, the relative values of $p_k$ and $q_k$ are respectively called a relative $p_k$ (or $rp_k$) and a relative $q_k$ (or $rq_k$). In one embodiment, $rp_k$ = $p_k/C$ and $rq_k$ = $q_k/C$, and $rp_k$ ($rp_a$, ..., $rp_b$) with k being in a range from a to b and/or $rq_k$ ($rq_c$, ..., $rq_d$) with k being in a range from c to d (here, a, b, c, and d are integers independently selected from the group consisting of 1 to n, a < b, c < d, and n is as defined above) are obtained. Here, C is a function with respect to time or the number of measurement points, and may be, for example, VmaxT, APTT or an equivalent in the number of measurement points, any $p_k$ and $q_k$, or an after-mentioned weighted average time $vT(S_k)$. A value in which the difference between the obtained ($rp_a$, ..., $rp_b$) and/or ($rq_c$, ..., $rq_d$), and ($rp_a$, ..., $rp_b$) and/or ($rq_c$, ..., $rq_d$) of the reference specimen is reflected is adopted as the parameter. For example, the slope $\delta$ of the linear regression line of a plot of ($rp_a$, ..., $rp_b$) and/or ($rq_c$, ..., $rq_d$) of the test specimen against ($rp_a$, ..., $rp_b$) and/or ($rq_c$, ..., $rq_d$) of the reference specimen can be adopted as the parameter. The parameter is obtained by quantification which allows the degree of the difference in the progression of the coagulation reaction of the specimen containing the FVIII-substituting substance to be exhibited, using $p_k$ and $q_k$ which represent the progression of the coagulation reaction (coagulation reaction rate curve), with reference to the progression of the specimen containing no FVIII-substituting substance. The size of the peak width of V of the specimen containing the FVIII-substituting substance, or the asymmetric degree of the peak shape is reflected in this parameter.

As described above, the blood coagulation ability of the test specimen is reflected in the aforementioned $p_k$, $q_k$, or parameters calculated therefrom, which suggests presence of the FVIII-substituting substance in the test specimen. Using these parameters can detect the presence or absence of the FVIII-substituting substance in the test specimen.

4.3) Parameter based on weighted average of V

In one embodiment, the parameter for analyzing the blood coagulation ability of the test specimen is a parameter based on the weighted average of V. Specifically, they may be the kurtosis ($vAB(S_k)$) in a predetermined region below V(i) (i denotes time or the number of measurement points) and the rate over time ($vTB(S_k)$) of the following equation.

$$vAB(S_k) = vH(S_k)/vB(S_k)$$

$$vTB(S_k) = vB(S_k)/VmaxT$$

where

$$vH(Sk) = \frac{\sum_{i=pk}^{qk}\left(i \times V(i)\right)}{\sum_{i=pk}^{qk} i}$$

$p_k$, $q_k$, $S_k$, and $vB(S_k)$ are as defined in the aforementioned 4.2).
That is,

$$V(p_k - D) < Vmax \times S_k \ (\%) \leq V(p_k),$$

and

$$V(q_k) \geq Vmax \times S_k \ (\%) > V(q_k + D),$$

where $p_k$ and $q_k$ represent time or the number of measurement points, $p_k \leq VmaxT \leq q_k$, and VmaxT satisfies V(VmaxT) = Vmax,
D denotes measurement interval,
$0 < S_k \leq 100$, preferably, $0.5 \leq S_k \leq 99$, and more preferably, $5 \leq S_k \leq 50$.
k denotes an integer ranging from 1 to n,
n denotes an integer ranging, preferably, from 5 to 50, and more preferably, from 10 to 30,
$vB(S_k)$ denotes an interval from $p_k$ to $q_k$ (time or the number of measurement points).

Note that a parameter, such as APTT or the equivalent in the number of measurement points, or the weighted average time $vT(S_k)$ described below, can be used as the denominator of an equation for calculating $vTB(S_k)$, instead of VmaxT. Note that $vT(S_k)$ can be obtained as a function of time or a function of the number of measurement points, depending on whether i indicates time or the number of measurement points.

$$vT(Sk) = \frac{\sum_{i=pk}^{qk}(i \times V(i))}{\sum_{i=pk}^{qk} V(i)}$$

$vH(S_k)$ corresponds to the Y-coordinate of the weighted average point of the region satisfying $V(i) \geq Vmax \times S_k$ (%) below V(i). $vAB(S_k)$ and $vTB(S_k)$ respectively represent the kurtosis of the region and the rate over time.

4.4) Parameter based on relative value of V

In one embodiment, the relativized maximum of the first derivative curve (hereinafter, corrected Vmax) is determined. For example, R is relativized such that the maximum is a predetermined value (for example, 100), and subsequently, the maximum of the first derivative curve obtained by the first derivative thereof is determined as the corrected Vmax. Alternatively, the corrected Vmax is also obtained by multiplying Vmax by the correction coefficient (= the maximum of predetermined value/R). The corrected Vmax may be used as the parameter for analyzing the blood coagulation ability of the test specimen.

4.5) Other parameters

A value obtained by multiplying or dividing the aforementioned parameter by a value about the coagulation reaction time or the number of measurement points, for example, VmaxT or $vT(S_k)$, or APTT or their equivalent in the number of measurement points, may be used as the parameter for analyzing the blood coagulation ability of the test specimen in the present invention. Such parameters include [corrected Vmax $\times$ VmaxT], [corrected Vmax $\times$ APTT], and [$vAB(S_k) \times$ VmaxT]. The parameter in the present invention may be a parameter about time or a parameter about the number of measurement points, depending on whether i, $p_k$, and $q_k$ are time or the number of measurement points, and in a case of multiplying or dividing the parameter as described above, it is preferable that the parameter about time be multiplied or divided by a function of time, and the parameter about the number of measurement points be multiplied or divided by a function of the number of measurement points.

4.6) Standard deviation index (SDI)

A value obtained by converting the unit of each of the parameters described in aforementioned 4.2) to 4.5) into the standard deviation index (SDI) with respect to a reference value can be used as the parameter for analyzing the blood coagulation ability of the test specimen in the present invention. Specifically, SDI obtained by the following equation using the statistics of the specimen containing no FVIII-substituting substance (that is, a specimen which contains no FVIII-substituting substance and has no prolonged coagulation time; hereinafter also called a non-administered (ND) specimen) can be adopted as the parameter. More preferably, the absolute value of SDI (|SDI|) obtained by the following equation can be adopted as the parameter:

|SDI| = |{(parameter value calculated from the test specimen) - (average of parameter values calculated from ND specimen group)}/(standard deviation of parameter values calculated from the ND specimen group)|.

5. Detection of specimen containing FVIII-substituting substance

As described in the after-mentioned Example, the values of the aforementioned parameters calculated in the present invention for analyzing the blood coagulation ability, pre-post average difference, all CV, qp ratio, $vB(S_k)$, $vAB(S_k)$, $vTB(S_k)$, parameters about the relative values of $p_k$ and $q_k$, corrected Vmax, their SDI and the like showed different tendencies between specimens containing the FVIII-substituting substance obtained from patients to whom the FVIII-substituting substance (emicizumab) had been administered, and specimens containing no FVIII-substituting substance. Based on these parameters, the specimen containing the FVIII-substituting substance can be detected.

In analysis of the blood coagulation ability by the present invention, examples of parameters used to detect the specimen containing the FVIII-substituting substance include at least one selected from the group consisting of the aforementioned pre-post average difference, all CV, qp ratio, $vB(S_k)$, $vAB(S_k)$, $vTB(S_k)$, parameters about relative values of $p_k$ and $q_k$, and corrected Vmax. The parameters used to detect the specimen containing the FVIII-substituting substance also include values obtained by multiplying or dividing the parameters by values about the coagulation reaction time or the number of measurement points, for example, VmaxT, APTT, or its equivalent in the number of measurement points, or $vT(S_k)$, for example, [corrected Vmax $\times$ VmaxT], [corrected Vmax $\times$ APTT], [$vAB(S_k)$ $\times$ VmaxT], and [$vAB(S_k)$ $\times$ APTT]. Alternatively, the SDI of each of the aforementioned parameters may also be an example of the parameter used to detect the specimen containing the FVIII-substituting substance. In the analysis of the blood coagulation ability by the present invention, these parameters may be any one kind of them, or two or more kinds may be combined and used. Preferably, at least one selected from the group consisting of the pre-post average difference, all CV, qp ratio, and $vTB(S_k)$, parameters about relative values of $p_k$ and $q_k$, and SDI of any of these parameters may be used.

In the detection of the specimen containing the FVIII-substituting substance based on the parameter, for example, in the cases where the parameters are about the pre-post average difference, all CV, qp ratio, $vB(S_k)$, $vTB(S_k)$, and relative values of $p_k$ and $q_k$ and where the parameter calculated from the test specimen is greater or equal to a predetermined detection threshold (hereinafter, also simply called "threshold") or greater than the threshold, the test specimen can be detected as the specimen containing the FVIII-substituting substance. For example, in the cases where the parameters are $vAB(S_k)$, [$vAB(S_k)$ $\times$ VmaxT], [$vAB(S_k)$ $\times$ APTT], corrected Vmax, [corrected Vmax $\times$ VmaxT], and [corrected Vmax $\times$ APTT] and where the parameter calculated from the test specimen is less than or equal to the threshold, or less than the threshold, the test specimen can be detected as the specimen containing the FVIII-substituting substance.

A value which can distinguish the specimen containing the FVIII-substituting substance, and the specimen containing no FVIII-substituting substance(ND specimen) from each other can be set as the threshold. For example, in the cases where the parameters are about the pre-post average difference, all CV, qp ratio, $vTB(S_k)$, and relative values of $p_k$ and $q_k$, the maximum of the parameter calculated from the ND specimen group, or average + K $\times$ standard deviation (SD) (preferably, K ranges from 1.5 to 3.4) can be set as the threshold. On the other hand in the cases where the parameters are [$vAB(S_k)$ $\times$ VmaxT], [$vAB(S_k)$ $\times$ APTT], [corrected Vmax $\times$ VmaxT], and [corrected Vmax $\times$ APTT], the minimum of the parameter calculated from the non-administered specimen group, or average - K $\times$ standard deviation (SD) (preferably, K ranges from 1.3 to 2.3) can be set as the threshold.

The specimen containing the FVIII-substituting substance and the ND specimen can be distinguished from each other with reference to a two-dimensionally changing threshold (threshold line) instead of a threshold which has a fixed value. For example, if a value of the test specimen plotted on (APTT, $vB(S_k)$) two-dimensional plane is above the threshold line (for example, [$vB(S_k) = a \times APTT + b$]; a and b are any coefficients), the test specimen can be detected as the specimen containing the FVIII-substituting substance. For example, if a value of the test specimen plotted on (APTT, $vAB(S_k)$) plane

or (APTT, corrected Vmax) plane is below the threshold line (for example, $[vAB(S_k) = a \times APTT + b]$ or [corrected Vmax = a $\times$ APTT + b]; a and b are any coefficients), the test specimen can be detected as the specimen containing the FVIII-substituting substance. The threshold line can be obtained by, for example, a procedure as described in after-mentioned Example 1.

In the case where the parameter is the SDI of each parameter described above, the threshold can be set based on the SDI of the parameter preliminarily calculated from the specimen containing the FVIII-substituting substance group or the ND specimen group. For example, the threshold for |SDI| described above can be set as follows: what has a value closest to the distribution region of the ND specimen group is extracted from among the parameters calculated from the specimen containing the FVIII-substituting substance group, and its |SDI| is obtained by the aforementioned equation; meanwhile, what has a value closest to the distribution region of the FVIII-substituting substance group is extracted from among the parameters calculated from the ND specimen group, and its |SDI| is obtained by the aforementioned equation; and an intermediate value between the former |SDI| and the latter |SDI| can be set as the threshold (see after-mentioned Example 9). Alternatively, instead of the intermediate value, any value which can distinguish the distributions of the former |SDI| and the latter |SDI| from each other may be set as the threshold. If |SDI| calculated from the test specimen is greater than the threshold, the test specimen can be detected as the specimen containing the FVIII-substituting substance.

Two or more of the parameters can be used as the reference for the detection. In this case, a threshold (or a threshold line) is set for each parameter, and it is determined whether each parameter calculated from the test specimen satisfies the reference based on the corresponding threshold (or the threshold line). In one example, if all the two or more parameters satisfy the threshold, the test specimen is detected as the specimen containing the FVIII-substituting substance. In another example, if at least one of the two or more parameters satisfies the threshold, the test specimen is detected as the specimen containing the FVIII-substituting substance.

The specimen detected as the specimen containing the FVIII-substituting substance is a specimen derived from a patient with an abnormality of the blood coagulation ability to which the FVIII-substituting substance has been administered, and the patient is estimated as a patient with a high bleeding risk in surgery or the like. Clinically, emicizumab, which is a drug for suppressing the bleeding tendency of hemophilia A, has been known as an FVIII-substituting substance. Consequently, the specimen containing the FVIII-substituting substance detected by the method of the present invention can be estimated as a specimen derived from a patient with hemophilia A to whom emicizumab has been administered.

As for the aforementioned detection reference, those skilled in the art can understand that as long as the specimen containing the FVIII-substituting substance can be detected, references which are "greater than the threshold" and "the greater than or equal to the threshold" can be interchangeably used, and likewise, references which are "less than the threshold" and "less than or equal to the threshold" can be interchangeably used.

**6.** Application to other coagulation reaction measurement methods

The method of the present invention has thus been described above using, as the example, the case of coagulation reaction measurement based on the scattered light intensity. However, those skilled in the art can apply other coagulation reaction measurement methods (for example, coagulation reaction measurement based on the transmittance, absorbance, viscosity and the like) to the coagulation reaction measurement in the present invention. Accordingly, such applications are encompassed by the scope of the present invention. For example, in the reaction R(i) represented by an inverted sigmoidal coagulation reaction curve based on the transmittance (transmittance light intensity), the positive and the negative are reversed with respect to that described based on the scattered light intensity. It is obvious to those skilled in the art that in such a case, in the aforementioned method of the present invention, the signs of R(i) and V(i) are reversed, the minimum Vmin is determined instead of the maximum Vmax of V, and $p_k$ and $q_k$ are respectively determined as points which reach $X_k$ before or after V(i) reaches Vmin.

7. Program and device

The method for analyzing the blood coagulation ability in the present invention can be automatically performed using a computer program. Consequently, an aspect of the present invention is a program for performing the aforementioned method for analyzing the blood coagulation ability in the present invention. A series of steps of the aforementioned method of the present invention can be automatically performed by an automatic analysis device. Consequently, an aspect of the present invention is a device for performing the aforementioned method for analyzing the blood coagulation ability in the present invention. Execution of the method of the present invention by the device can be controlled by the program in the present invention. Another aspect of the present invention is a system for performing the method for analyzing the blood coagulation ability in the present invention. The system includes the device or the program for performing the aforementioned method for analyzing the blood coagulation ability in the present invention.

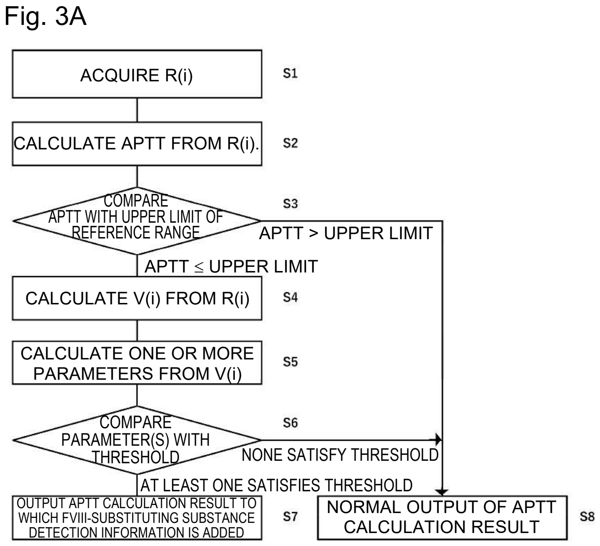

An example of a blood coagulation analyzing process by the present invention executed under control of the program of the present invention is described below and in Figure 3A.

S1: Acquire R(i) from (freely) measured coagulation reaction data.
S2: Calculate APTT from R(i).
S3: Go to S4 if APTT is not prolonged, and to S8 if APTT is prolonged.
S4: Calculate V(i) from R(i).
S5: Calculate one or more parameters from V(i).
S6: Compare the parameter(s) with threshold.

→ If at least one parameter satisfies the threshold, go to S7.
→ If no parameter satisfies the threshold, go to S8.

S7: Output detection of APTT and the specimen containing the FVIII-substituting substance.
S8: Output APTT.

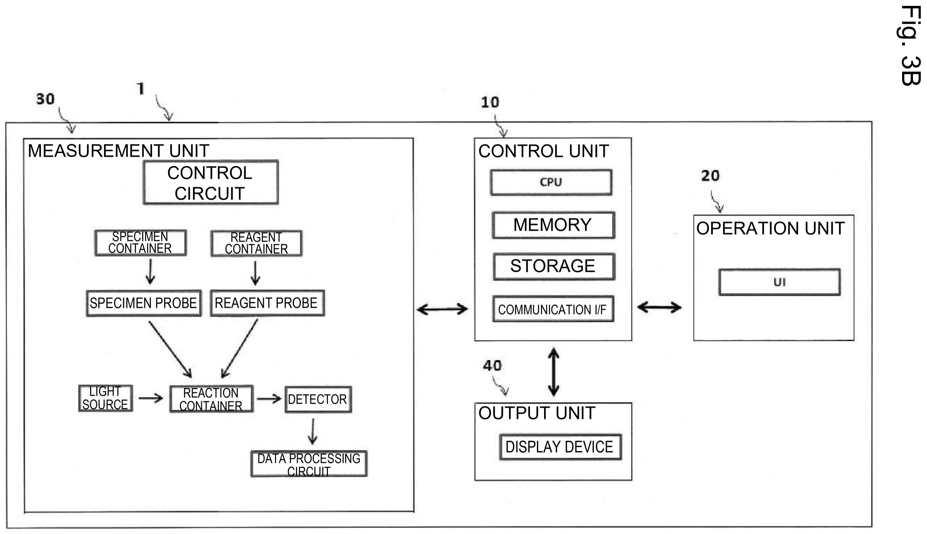

One embodiment of the device in the present invention is described below. The one embodiment of the device of the present invention is an automatic analysis device 1 as illustrated in Figure 3B. The automatic analysis device 1 includes a control unit 10, an operation unit 20, a measurement unit 30, and an output unit 40.

The control unit 10 controls the entire operation of the automatic analysis device 1. The control unit 10 may be made up of a computer (for example, a personal computer). The control unit 10 includes a CPU, a memory, a storage, a communication interface (I/F) and the like, and can perform processing of commands from the operation unit 20, control of the operation of the measurement unit 30, storing and data analysis of measured data received from the measurement unit 30, storing of an analysis result, control of outputting of the analysis result by the output unit 40 and the like. Furthermore, the control unit 10 may be connected to other devices, such as an external medium, and a host computer. Note that in the control unit 10, the computer which controls the operation of the measurement unit 30, and the computer which analyzes data measured by the unit 30 may be identical to or different from each other.

The operation unit 20 acquires input from an operator, and transmits the obtained input information to the control unit 10. For example, the operation unit 20 includes an user interface (UI), such as a keyboard and a touch panel. Under control of the control unit 10, the output unit 40 outputs: a detection result of the specimen containing the FVIII-substituting substance, and coagulation reaction data measured by the measurement unit 30 as required; and R, V, and a parameter for analyzing the blood coagulation ability based thereon, and an analysis result, such as the coagulation time (APTT) as required. For example, the output unit 40 includes a display device, such as a display.

The measurement unit 30 executes the series of operations for analyzing the blood coagulation, and acquires measured data of the coagulation reaction of a sample which contains the blood specimen. The measurement unit 30 includes various instruments and analysis modules required to analyze the blood coagulation, for example, a specimen container which stores the blood specimen, a reagent container which stores a reagent for a test, a reaction container for the reaction between the specimen and the reagent, a probe for dispensing the blood specimen and the reagent into the reaction container, a light source, a detector for detecting scattered light or transmitted light from the sample in the reaction container, a data processing circuit which transmits data from the detector to the control unit 10, and a control circuit which controls the operation of the measurement unit 30 in response to an instruction of the control unit 10. Alternatively, in a case where V is acquired using known coagulation reaction data, and the parameter is calculated, the measurement unit 30 is not required.

The control unit 10 performs analysis of the coagulation reaction of the specimen, based on the coagulation reaction data. This analysis may include acquisition of the aforementioned R and V, calculation of APTT, calculation of various parameters, and detection of the specimen containing the FVIII-substituting substance using the parameter. Alternatively, R and V may be created by the control unit 10 based on the measured data from the measurement unit 30, or may be created by another device, for example, the measurement unit 30 and then be transmitted to the control unit 10. The control unit 10 may store a threshold used to detect the specimen containing the FVIII-substituting substance, or the control unit 10 may import the threshold stored in an external device or on a network, for the sake of the detection.

This analysis described above can be executed by the program for performing the method of the present invention. Consequently, the control unit 10 may include the program for performing the method for analyzing the blood coagulation ability in the present invention.

The analysis result in the control unit 10 is transmitted to the output unit 40 and is output. The output may be in any form, such as being displayed on a screen, being transmitted to a host computer, or being printed. Output information from the output unit can include R or V, APTT, a parameter for analyzing the blood coagulation ability, and a detection result of the specimen containing the FVIII-substituting substance. Furthermore, the output information may include other information obtained by APTT measurement (detection of an initial response, possibility of a chyle specimen and the like). The kind of output information from the output unit can be controlled by the program of the present invention.

Examples

The present invention is described in further detail below with reference to Examples. However, the present invention is not limited to these Examples.

1. Method

1.1) Specimen

- Control specimen group (NE): plasma (n = 30) with APTT being within the reference standard range (from 24 to 39 seconds) of Coagpia APTT-N (manufactured by Sekisui Medical Co., Ltd.).
- Test specimen group (E1): plasma (n = 54) obtained from a patient with hemophilia A to whom emicizumab had been administered. The emicizumab concentration ranged from 12.2 to 79.2 μg/mL.
- Test specimen group (E2): plasma (n = 15) obtained from a patient with hemophilia A who had an antibody (inhibitor) against the coagulation factor VIII (FVIII) and to whom emicizumab had been administered. The emicizumab concentration ranged from 12.0 to 64.0 μg/mL, and the inhibitor potency ranged from 0.8 to 202 BU/mL.

1.2) Coagulation reaction measurement

Coagpia APTT-N (manufactured by Sekisui Medical Co., Ltd.), which is a reagent for APTT measurement, was used as reagent for measurement, and Coagpia APTT-N calcium chloride solution (manufactured by Sekisui Medical Co., Ltd.) was used as a calcium chloride solution. The coagulation reaction of a sample containing a specimen was measured using the blood coagulation automatic analysis device CP3000 (manufactured by Sekisui Medical Co., Ltd.). 50μL of the specimen was heated in a cuvette at 37°C for 45 seconds, 50μL of the reagent for measurement at approximately 37°C was subsequently added, and after 171 seconds elapsed, 50μL of the calcium chloride solution was added, thus initiating the coagulation reaction. The reaction was performed at 37°C. In the measurement of the coagulation reaction, the cuvette was irradiated with light of a wavelength of 660 nm from a light source which is an LED, and the scattered light intensity of 90-degree side scattered light was measured at 0.1-second intervals. The measurement time was 360 seconds.

1.3) Acquisition of R(i) and V(i)

To measured data of the coagulation reaction from each specimen, after a smoothing process including denoising was applied, a zero adjustment process was applied so as to allow the scattered light intensity at the measurement initiation time point to be zero, and the coagulation reaction R(i) (i is time from the coagulation reaction initiation) was created. From R(i), the first derivative (coagulation reaction rate) V(i) was calculated. The maximum Vmax of V(i) of each specimen, and the time VmaxT at which V(i) reached the maximum Vmax were calculated.

1.4) APTT calculation

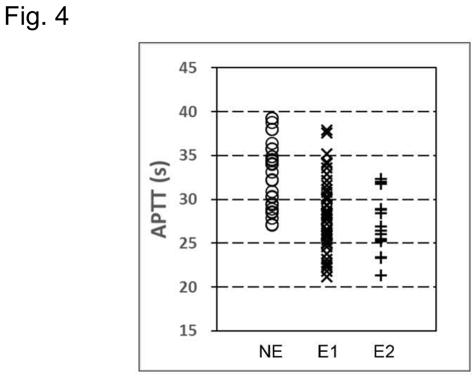

APTT of each specimen was calculated by the percent detection method. That is, a time point at which R(i) reached maximum Rmax within the measurement time was determined as a coagulation reaction end point E, and a time point at which R(i) reached 50% of R(E) was adopted as APTT. Figure 4 illustrates APTT of NE, E1, and E2. APTT ranged from 27.1 to 39.3 seconds for NE, from 21.2 to 37.9 seconds for E1, and from 21.3 to 32.3 seconds for E2. The APTT distribution regions of E1 and E2 overlap the distribution region of NE. E1 and E2 exhibited APTT shorter than the lower limit (24 seconds) of the reference standard range in some cases. This result showed that the emicizumab-administered groups (E1 and E2), and the non-emicizumab-administered group (NE) cannot be distinguished from each other based only on APTT.

1.5) Creation of corrected reaction rate curve

To compare the shapes of rate curves of the specimens, a corrected reaction rate curve was created in the next procedure. The curve was relativized such that the maximum of V(i) was 100, and shifted in the temporal axis direction such that the time of reaching the maximum was 40 seconds, thus obtaining a corrected reaction rate curve. To display this corrected reaction rate curve, the abscissa axis name was defined as the corrected time, and the ordinate axis name was defined as the corrected reaction rate.

1.6) Calculation of corrected Vmax

By multiplying Vmax of each specimen by a coefficient F (= 100/Rmax), and the corrected Vmax (= Vmax $\times$ F) was calculated.

1.7) Calculation of parameter about V(i)

The parameter about V(i) of each specimen was calculated. It was defined that $X_k$ = Vmax $\times$ $S_k$ (%) (k is an integer from 1 to 20, and $S_k$ increases from 3% to 98% in increments of 5%; that is, $S_1$ = 3%, and $S_{20}$ = 98%). 20 $p_k$ ($p_1$, ..., $p_{20}$) including minimums with i satisfying V(i) $\geq X_k$ with each k at time points before VmaxT, and 20 $q_k$ ($q_1$, ..., $q_{20}$) including maximums with i satisfying V(i) $\geq X_k$ with each k at time points after VmaxT were acquired (see Figure 2). From the obtained $p_k$ and $q_k$, the following parameters were calculated.

Pre-Ave: the average of $p_k$ (k is any integers selected from a range from 1 to 20),
Post-Ave: the average of $q_k$ (k is as described above),
All-Ave: the average of $p_k$ and $q_k$,
Pre-post average difference: (post-Ave - pre-Ave)/all-Ave,
CV: coefficient of variation (%) about $p_k$ and $q_k$,
qp ratio: $q_k/p_k$,

$$\texttt{kurtosis vAB(S_k) = vH(S_k)/vB(S_k),}$$

or

$$\texttt{vAB(S_k) = vH(S_k)/vB(S_k) × VmaxT,}$$

or

$$\texttt{vAB(S_k) = vH(S_k)/vB(S_k) × APTT,}$$

Rate over time vTB($S_k$) = vB ($S_k$) /VmaxT.
Note that vH($S_k$) and vB($S_k$) are as follows
vB ($S_k$): $q_k$ - $p_k$ + 0.1 (seconds)

$$vH(Sk) = \frac{\sum_{i=pk}^{qk}\left(i \times V(i)\right)}{\sum_{i=pk}^{qk} i}$$

($p_k$, $q_k$, $S_k$, and k are as described above)

Hereinafter, parentheses on both the sides in each parameter name are sometimes abbreviated for representation. For example, vB(30) is sometimes represented as vB30.

2. Coagulation reaction

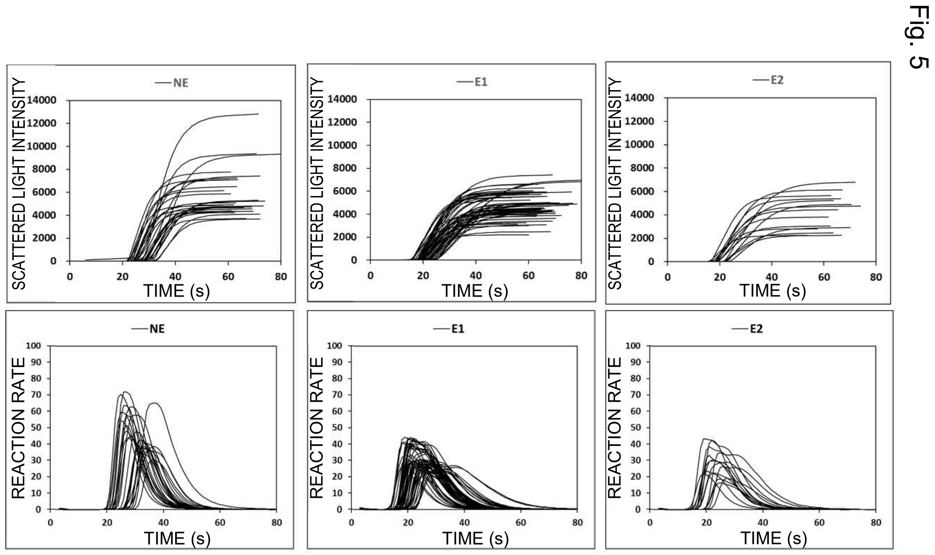

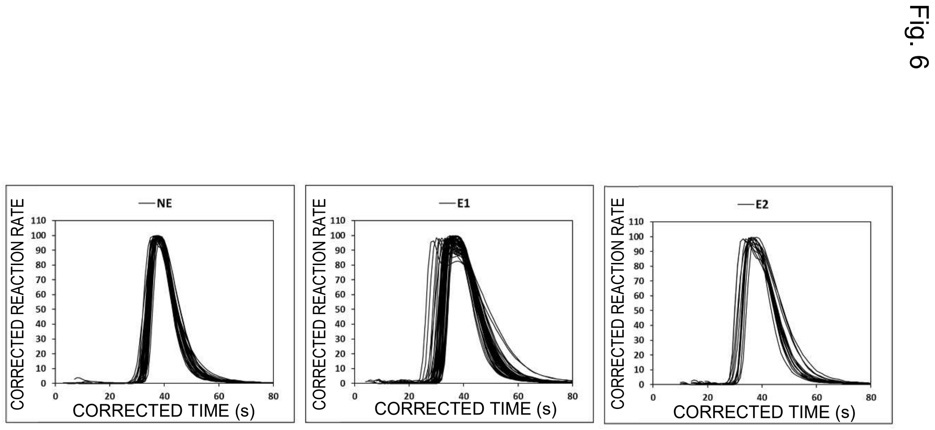

Figure 5 illustrates the coagulation reaction curves R(i) (zero-adjusted scattered light intensity) (upper), and the first derivative curve V(i) (lower) of NE, E1, and E2 plotted against the measurement time. E1 and E2, in comparison with NE, had tendencies where the rise time of the reaction curve was rapid but the increase of the curve was gradual. Figure 6 illustrates the corrected reaction rate curves of NE, E1, and E2 plotted against the corrected time. In comparison with NE, the corrected reaction rate curves of E1 and E2 had tendencies where the peak width was wide, and the peak shape was strongly asymmetric (the descent from the peak top was gradual).

3. Comparison of parameters between specimen groups

3.1) Comparative Example 1 Vmax

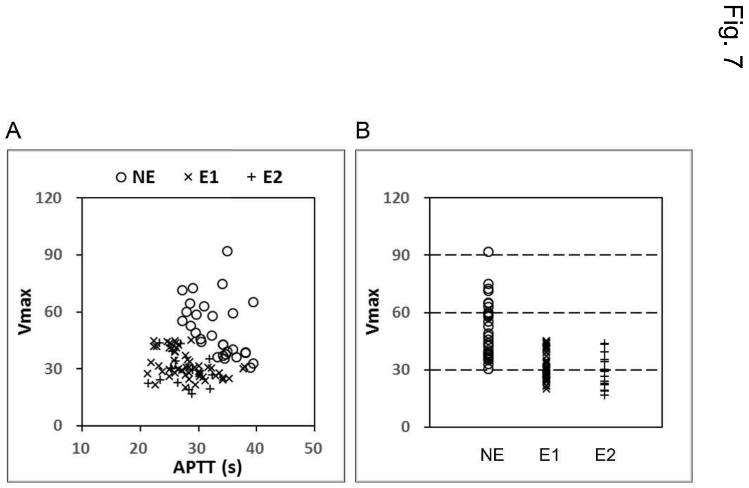

Figure 7 illustrates a distribution of the maximums Vmax of V(i) about NE, E1, and E2. Figure 7A illustrates a plot of Vmax against APTT of each specimen. Figure 7B illustrates a Vmax distribution of each specimen group. Vmax of E1 and E2 tended to be smaller than that of NE, but the distribution regions overlapped that of NE. The emicizumab-administered groups (E1 and E2), and non-administered group (NE) were unable to be distinguished from each other based only on Vmax.

3.2) Comparative Example 2 VmaxT

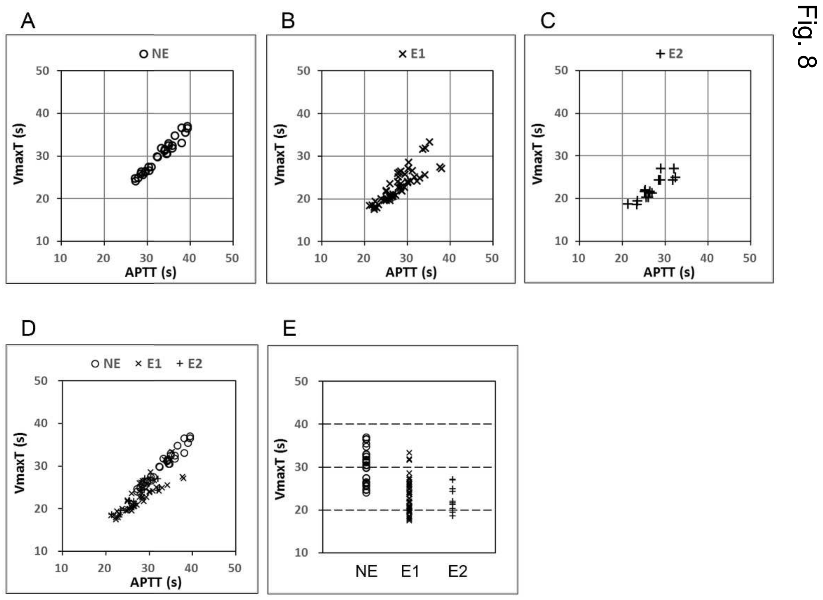

Figures 8A to 8D illustrate a plot of VmaxT against APTT about NE, E1, E2, and all specimens, respectively. Figure 8E illustrates the VmaxT distribution for each specimen group. While VmaxT of E1 and E2 tended to be smaller than that of NE, the distribution regions overlapped that of NE. The emicizumab-administered groups (E1 and E2), and non-administered group (NE) were unable to be distinguished from each other based only on VmaxT.

3.3) Example 1 corrected Vmax

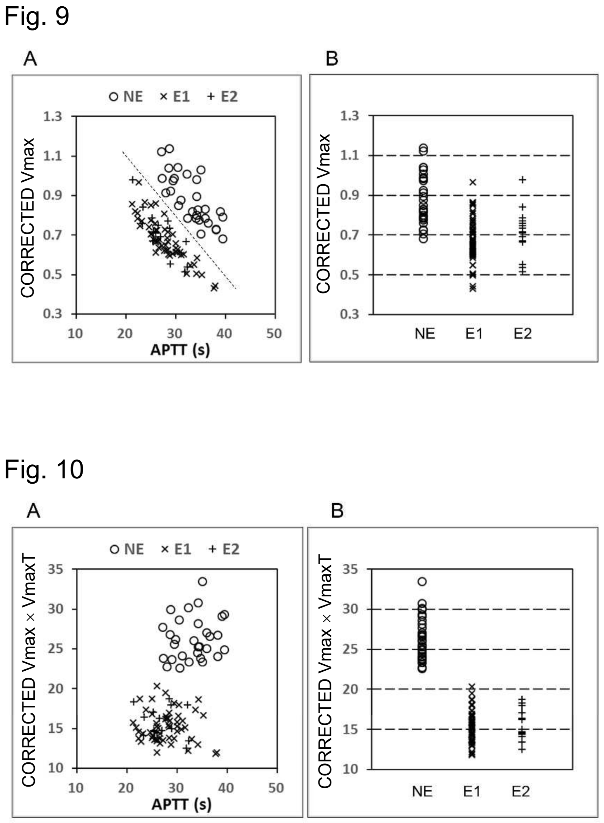

Figure 9 illustrates a corrected Vmax distributions for NE, E1, and E2. Figure 9A illustrates a plot of Vmax against APTT of each specimen. Figure 9B illustrates the corrected Vmax distribution of each specimen group. The dotted line in Figure 9A was obtained by the following procedure: 1) a regression line y = ax + b of the plot of the specimens of NE was obtained; 2) a plotted point (outermost point) which was closer to distributions of E1 and E2 than the regression line among the plotted points of NE, and was farthest in distance from the regression line was obtained; 3) a plotted point (closest point) which was closest to the regression line among the plotted points of E1 and E2 was obtained; and 4) A line which passed through the midpoint of a line connecting the outermost point and the closest point and was parallel to the regression line was calculated. It was shown that the distribution region of the corrected Vmax was different between the emicizumab-administered groups (E1 and E2) and the non-administered group (NE), and both the groups were able to be divided from each other by the dotted line of Figure 9A, the dotted line obtained by the procedure can be used as a two-dimensional threshold (threshold line) distinguishing the emicizumab-administered groups (E1 and E2) and the non-administered group (NE). It was shown that by setting such a threshold line, the emicizumab-administered groups (E1 and E2) and the non-administered group (NE) could be distinguished from each other based on APTT and the corrected Vmax.
Figure 10A shows plots of [corrected Vmax × VmaxT] of the specimens of NE, E1, and E2 against APTT. Figure 10B illustrates a distribution of [corrected Vmax × VmaxT] of each specimen group. It was shown that the distribution region of [corrected Vmax × VmaxT] was different between the emicizumab-administered groups (E1 and E2) and the non-administered group(NE), and the emicizumab-administered groups (E1 and E2) and the non-administered group (NE) could be distinguished from each other based on the value.

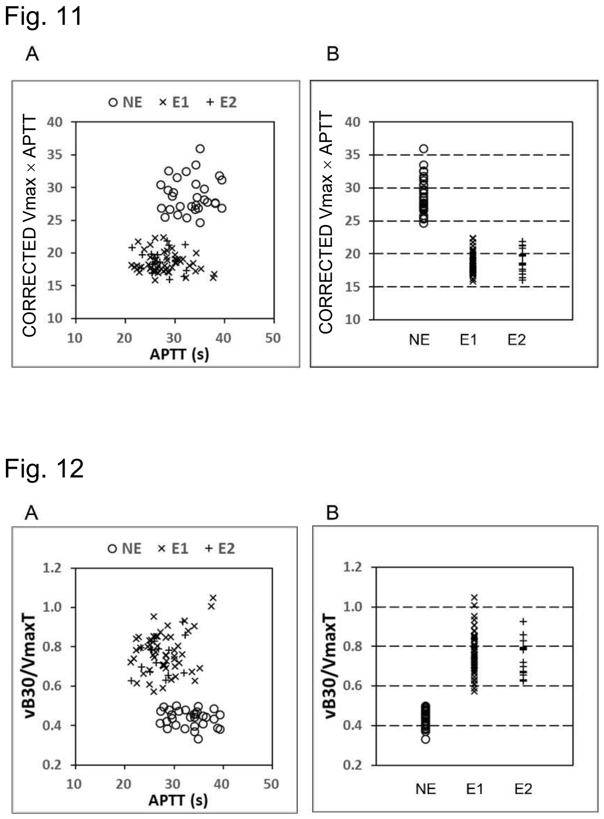

Figure 11A illustrates plots of [corrected Vmax × APTT] of the specimens of NE, E1, and E2 against APTT. Figure 11B illustrates a distribution of [corrected Vmax × APTT] of each specimen group. It was shown that the distribution region of [corrected Vmax × APTT] was different between the emicizumab-administered groups (E1 and E2) and the non-administered group(NE), and the emicizumab-administered groups (E1 and E2) and the non-administered group (NE) could be distinguished from each other based on the value. The results in Figures 10 and 11 showed that using the value obtained by multiplying the corrected Vmax by the parameter about the reaction time, such as VmaxT or APTT, could distinguish the emicizumab-administered groups (E1 and E2) and the non-administered group (NE) from each other with reference to a specific threshold without the need to set a two-dimensional threshold (threshold line).

3.4) Example 2 vTB($S_k$)

Figure 12A illustrates plots of vTB(30) of the specimens of NE, E1, and E2 against APTT. Figure 12B illustrates a distribution of vTB(30) of each specimen group. In the diagram, vTB(30) is represented by vB (30) /VmaxT. It was shown that the distribution region of vTB(30) was different between the emicizumab-administered groups (E1 and E2) and the non-administered group(NE), and the emicizumab-administered groups (E1 and E2) and the non-administered group (NE) could be distinguished from each other based on the value.

3.5) Example 3 vAB($S_k$)

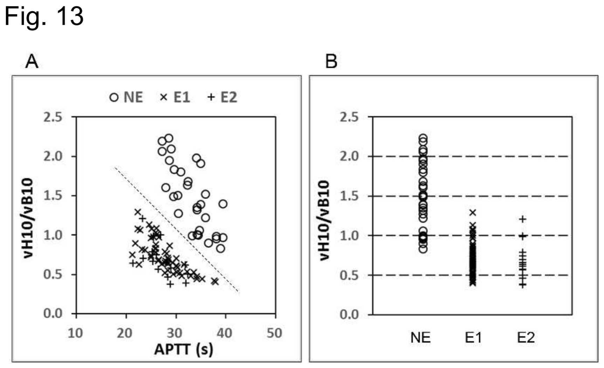

Figure 13A illustrates plots of vAB(10) = vH(10)/vB(10) of the specimens of NE, E1, and E2 against APTT. Figure 13B illustrates a distribution of vAB(10) of each specimen group. In the diagram, vAB(10) is represented by vH(10)/vB(10). The dotted line in Figure 13A was calculated in a manner similar to that of Example 1 (3.3 described above). The distribution region of vAB(10) was different between the emicizumab-administered groups (E1 and E2) and the non-administered group (NE). The groups could be divided by the dotted line in Figure 13A. It was shown that by setting such a two-dimensional threshold (threshold line), the emicizumab-administered groups (E1 and E2) and the non-administered group (NE) could be distinguished from each other based on APTT and the $vAB(S_k)$.

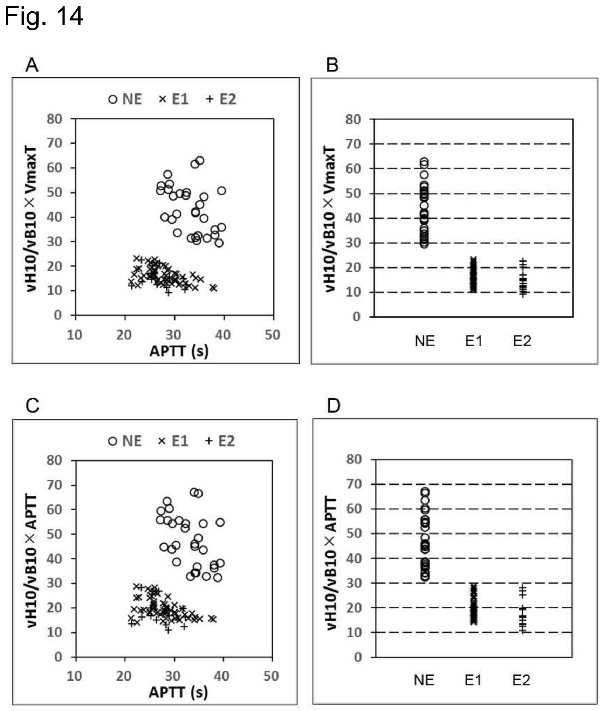

Figure 14A shows plots of [vAB(10) $\times$ VmaxT] of the specimens of NE, E1, and E2 against APTT. Figure 14B illustrates a distribution of [vAB(10) $\times$ VmaxT] of each specimen group. Figure 14C shows plots of [vAB(10) $\times$ APTT] of the specimens of NE, E1, and E2 against APTT. Figure 14D illustrates a distribution of [vAB(10) $\times$ APTT] of each specimen group. In the diagram, vAB(10) is represented by vH(10)/vB(10). [vAB(10) $\times$ VmaxT] and [vAB(10) $\times$ APTT] of the emicizumab-administered groups (E1 and E2) were lower than those of the non-administered group (NE). It was shown that using the value obtained by multiplying $vAB(S_k)$ by VmaxT or APTT could distinguish the emicizumab-administered groups (E1 and E2) and the non-administered group (NE) from each other with reference to a specific threshold without the need to set a two-dimensional threshold (threshold line).

3.6) Example 4 qp ratio

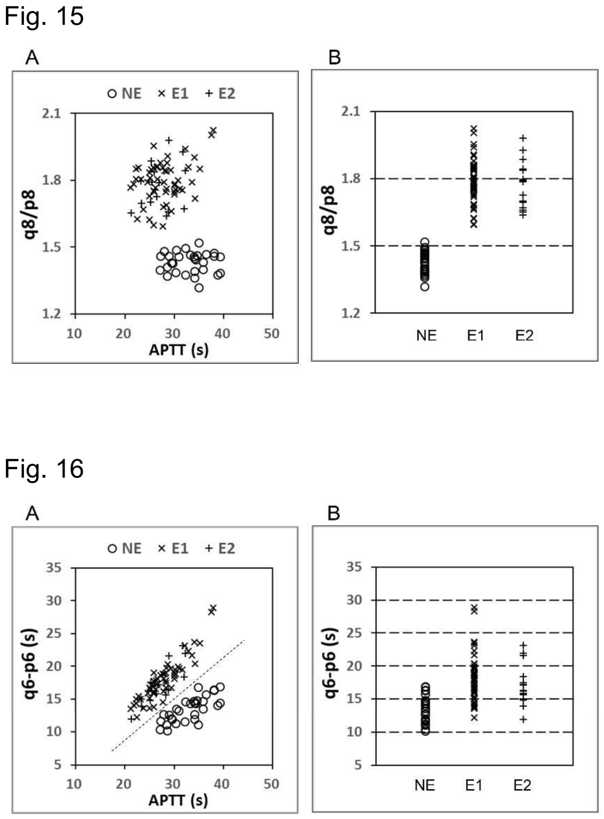

$p_k$ and $q_k$ with k = 8 were calculated, and qp ratio = $q_k/p_k$ was obtained. Figure 15A illustrates plots of qp ratio ($q_8/p_8$) of the specimens of NE, E1, and E2 against APTT. Figure 15B illustrates a distribution of qp ratio of each specimen group. It was shown that the distribution region of the pq ratio was different between the emicizumab-administered groups (E1 and E2) and the non-administered group(NE), and the emicizumab-administered groups (E1 and E2) and the non-administered group (NE) could be distinguished from each other based on the value. The size of the peak width of V(i) and the peak shape (asymmetric degree of the peak) in the emicizumab-administered groups (E1 and E2) against the non-administered group (NE) as shown in Figures 5 and 6 were estimated to be reflected in the distribution difference of the qp ratio between the emicizumab-administered groups (E1 and E2) and the non-administered group (NE).

3.7) Example 5 $vB(S_k)$

Figure 16A illustrates plots of $q_6 - p_6$ (vB(6)) of the specimens of NE, E1, and E2 against APTT. Figure 16B illustrates a distribution of $q_6 - p_6$ of each specimen group. The dotted line in Figure 16A was calculated in a manner similar to that of Example 1 (3.3 described above). The distribution region of $q_6 - p_6$ was different between the emicizumab-administered groups (E1 and E2) and the non-administered group (NE). The groups could be divided by the dotted line in Figure 16A. It was shown that by setting such a two-dimensional threshold (threshold line) like the dotted line, the emicizumab-administered groups (E1 and E2) and the non-administered group (NE) could be distinguished from each other based on APTT and $q_k - p_k$ ($vB(S_k)$). The size of the peak width of V(i) in the emicizumab-administered groups (E1 and E2) against the non-administered group (NE) as shown in Figures 5 and 6 were estimated to be reflected in the distribution difference of $vB(S_k)$ between the emicizumab-administered groups (E1 and E2) and the non-administered group (NE).

3.8) Example 6 pre-post average difference

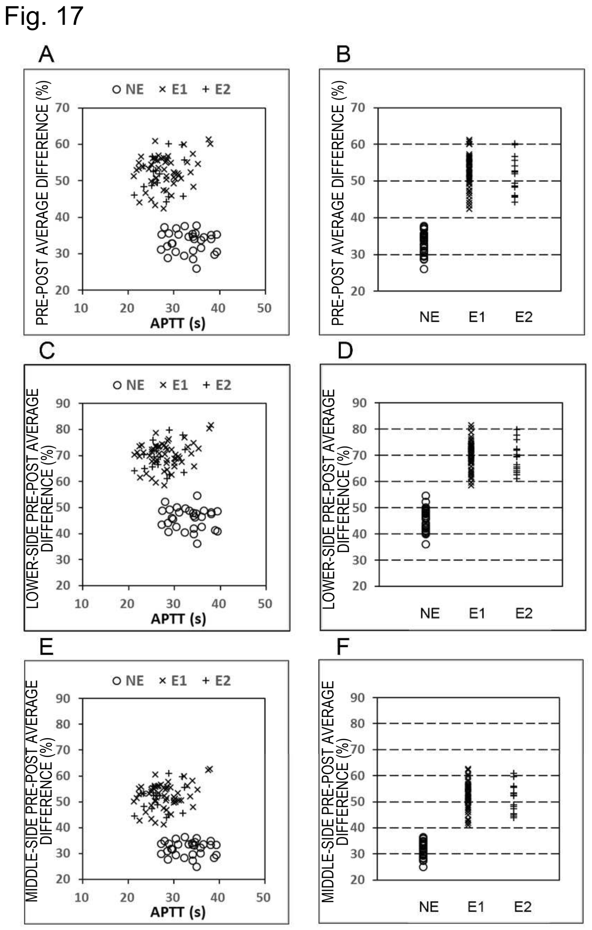

Figures 17A to 17F illustrate pre-post average difference of the specimens of NE, E1, and E2. Figures 17A, 17C, and 17E illustrate plots of the pre-post average difference against APTT. Figures 17B, 17D, and 17F illustrate a distribution of the pre-post average difference of each specimen group. Figures 17A and 17B illustrate the pre-post average difference about $p_k$ and $q_k$ detected at k = 1 to 20, that is, from the entire peak of V. Figures 17C and 17D illustrate the pre-post average difference (lower-side pre-post average difference) about $p_k$ and $q_k$ detected at k = 1 to 10, that is, from the lower side of the peak of V (lower-side pre-post average difference). Figures 17E and 17F illustrate the pre-post average difference about $p_k$ and $q_k$ detected at k = 6 to 15, that is, from the middle of the peak of V (middle-side pre-post average difference). In all the cases, it was shown that the distribution region of the pre-post average difference was different between the emicizumab-administered groups (E1 and E2) and the non-administered group(NE), and the emicizumab-administered groups (E1 and E2) and the non-administered group (NE) could be distinguished from each other based on the pre-post average difference. The peak shapes of V(i) in the emicizumab-administered groups (E1 and E2) against the non-administered group (NE) (asymmetric degree of the peak) as shown in Figures 5 and 6 were estimated to be reflected in the distribution difference of the pre-post average difference between the emicizumab-administered groups (E1 and E2) and the non-administered group (NE).

3.9) Example 7 all CV

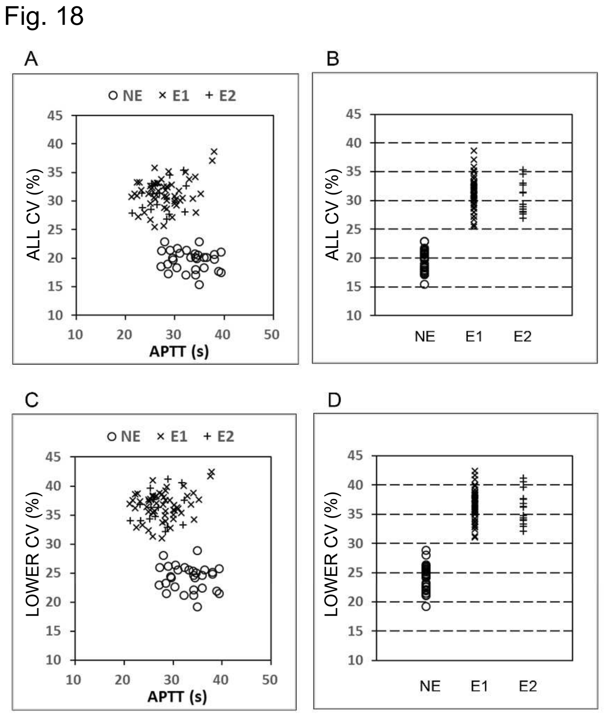

Figures 18A to 18D illustrate the CV of the $p_k$ and $q_k$ of the specimens of NE, E1, and E2. Figures 18A and 18C illustrate plots of CV against APTT. Figures 18B and 18D illustrate a distribution of CV of each specimen group. Figures 18A and 18B illustrate CV with $p_1$ to $p_{20}$ and $q_1$ to $q_{20}$ at k = 1 to 20 (all CV). Figures 18C and 18D illustrate CV with $p_1$ to $p_{10}$ and $q_1$ to $q_{10}$ at k = 1 to 10 (lower CV). It was shown that the distribution region of the CV of $p_k$ and $q_k$ was different between the emicizumab-administered groups (E1 and E2) and the non-administered group(NE), and the emicizumab-administered groups (E1 and E2) and the non-administered group (NE) could be distinguished from each other based on the CV of $p_k$ and $q_k$. The size of the peak width of V(i) in the emicizumab-administered groups (E1 and E2), and the peak shape (asymmetric degree of the peak) as shown in Figures 5 and 6 were estimated to be reflected in the distribution difference of the CV of $p_k$ and $q_k$ between the emicizumab-administered groups (E1 and E2) and the non-administered group (NE).

3.10) Example 8 ratio of $p_k$ and $q_k$ against VmaxT

The $p_k$ and $q_k$ of NE, E1, and E2 were compared. The $p_k$ and $q_k$ of the specimens of NE, E1, and E2 (k = 1 to 20 or k = 6 to 20) were divided by VmaxT, and the relative $p_k$ (= $p_k$/VmaxT) and relative $q_k$ (= $q_k$/VmaxT) were obtained. The NE specimen with APTT at the minimum (27.1 seconds) or the maximum (39.3 seconds) was used as the reference specimen. A linear regression line was obtained between the relative $p_k$ and relative $q_k$ data groups of the reference specimen, and the relative $p_k$ and relative $q_k$ data groups of each specimen.

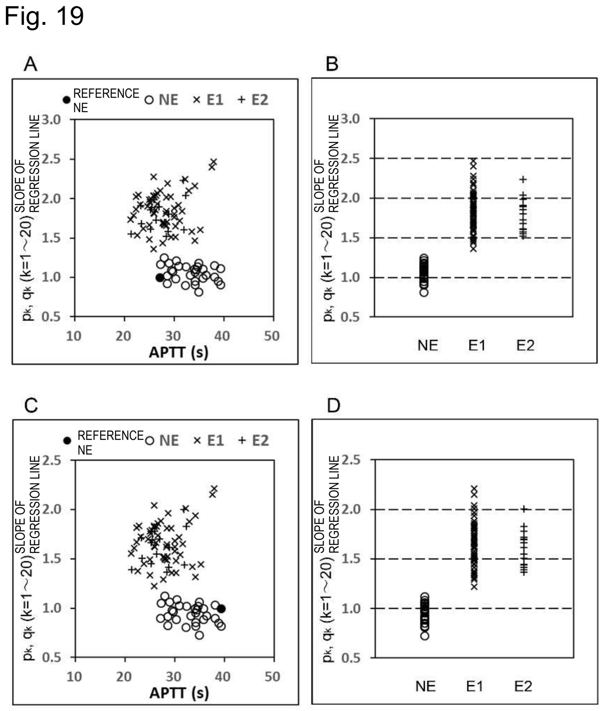

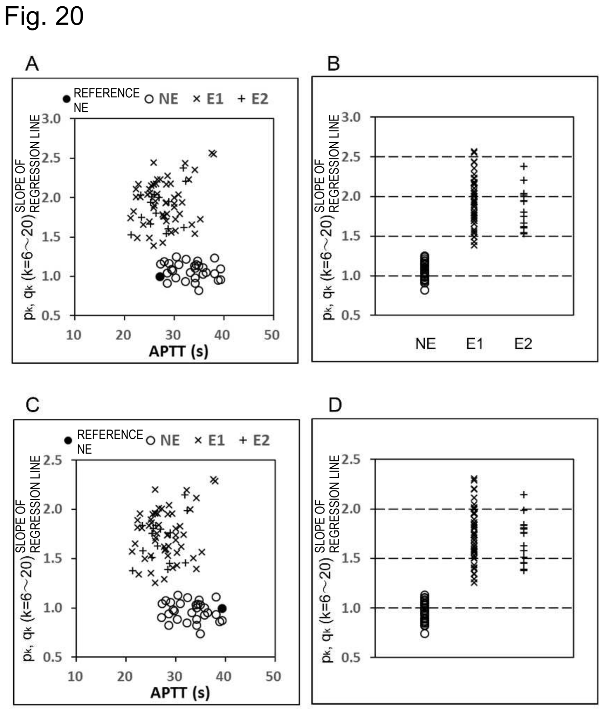

Figures 19A to 19D and Figures 20A to 20D illustrate distributions of the slopes of the regression lines of the specimens of NE, E1, and E2. Figure 19 illustrates the slope of the regression line of the relative $p_k$ and the relative $q_k$ (k = 1 to 20). Figure 20 illustrates the slope of the regression line of the relative $p_k$ and the relative $q_k$ (k = 6 to 20). As the reference specimen, the NE specimen with the minimum APTT was used in Figures 19A, 19B, 20A, and 20B, and the NE specimen with the maximum APTT was used in Figures 19C, 19D, 20C, and 20D. Figures 19A, 19C, 20A, and 20C illustrate plots of the slopes of the specimen against APTT. Figures 19B, 19D, 20B, and 20D illustrate a slope distribution of each specimen group. In Figures 19A, 19C, 20A, and 20C, the plotted points of the reference specimen (reference NE) are represented as solid circles •. In all the cases in Figures 19 to 20, the slopes of the regression lines of the emicizumab-administered groups (E1 and E2) tend to be greater than that of the non-administered group (NE). They showed that the emicizumab-administered groups and the non-administered group could be distinguished from each other based on this parameter.

3.11) Example 9 SDI of parameter

The proximity of the parameter (P) between the emicizumab-administered groups (Emi; including E1 and E2) and the non-administered group (NE) was evaluated. [corrected Vmax × VmaxT] illustrated in Figure 10, [corrected Vmax × APTT] illustrated in Figure 11, vTB(30) illustrated in Figure 12, [vAB(10) × VmaxT] and [vAB(10) × APTT] illustrated in Figure 14, $q_8/p_8$ illustrated in Figure 15, the pre-post average difference, lower-side pre-post average difference, and middle-side pre-post average difference illustrated in Figure 17, all CV and lower CV illustrated in Figure 18, and four regression line slopes respectively illustrated in Figures 19A and 19B, Figures 19C and 19D, Figures 20A and 20B, and Figures 20C and 20D were used as the parameter (P). The specimen with P being closest to Emi in NE (that is, P was the maximum or minimum) (NE proximal specimen), and the specimen with P being closest to NE in Emi (that is, P was the minimum or maximum) (Emi proximal specimen) were extracted. The absolute value of the standard deviation index (|SDI|) of P of each of the extracted two specimens with respect to the average of P in NE was calculated.

|SDI| = |{(NE proximal specimen or P of Emi proximal specimen) - (average of P in NE) }/(standard deviation of P in NE) |

Table 1 illustrates |SDI| of the NE proximal specimen and the Emi proximal specimen of each parameter. In the difference of |SDI| between the NE proximal specimen and the Emi proximal specimen, the fact that each parameter had a different distribution between Emi and NE was reflected, as illustrated in Examples 1 to 8. These results showed that using |SDI| of the parameter illustrated in Examples 1 to 8 could distinguish the emicizumab-administered group and the non-administered group from each other. For example, |SDI| of the parameter of the test specimen with respect to the reference value was obtained, and was compared with the threshold, thereby allowing detecting whether the test specimen is in the emicizumab-administered group or not. The average of the parameters of NE as described above can be used as the reference value. An intermediate value between |SDI| of the NE proximal specimen and |SDI| of the Emi proximal specimen as described above can be set as an example of the threshold.

[Table 1]

| Drawings | Parameter (P) | A: \|SDI\| of P of NE proximal specimen | B: \|SDI\| of P of Emi proximal specimen | B-A | Intermediate value between A and B |
|---|---|---|---|---|---|
| Fig. 10A | Corrected Vmax × VmaxT | 1.36 | 2.22 | 0.86 | 1.79 |
| Fig. 11A | Corrected Vmax × APTT | 1.53 | 2.38 | 0.86 | 1.96 |

(continued)

| Drawings | Parameter (P) | A: \|SDI\| of P of NE proximal specimen | B: \|SDI\| of P of Emi proximal specimen | B-A | Intermediate value between A and B |
|---|---|---|---|---|---|
| Fig. 12A | vTB(30) | 1.47 | 3.15 | 1.68 | 2.31 |
| Fig. 14A | vAB(10) × VmaxT | 1.42 | 2.07 | 0.65 | 1.74 |
| Fig. 14C | vAB(10) × APTT | 1.42 | 1.76 | 0.33 | 1.59 |
| Fig. 15A | q8/p8 | 1.88 | 3.45 | 1.57 | 2.66 |
| Fig. 17A | Pre-post average difference | 1.52 | 3.06 | 1.54 | 2.29 |
| Fig. 17C | Lower-side pre-post average difference | 2.10 | 3.04 | 0.94 | 2.57 |
| Fig. 17E | Middle-side pre-post average difference | 1.57 | 3.10 | 1.54 | 2.34 |
| Fig. 18A | All CV | 1.76 | 3.17 | 1.41 | 2.47 |
| Fig. 18C | Lower CV | 2.13 | 3.10 | 0.97 | 2.61 |
| Fig. 19A | Regression line slope | 1.81 | 2.85 | 1.04 | 2.33 |
| Fig. 19C | Regression line slope | 1.78 | 2.88 | 1.10 | 2.33 |
| Fig. 20A | Regression line slope | 1.68 | 2.94 | 1.26 | 2.31 |
| Fig. 20C | Regression line slope | 1.67 | 2.95 | 1.28 | 2.31 |

**Claims**

1. A method for analyzing a blood coagulation ability of a blood specimen, the method comprising:

   1) acquiring $V(i)$, which is a first derivative of a coagulation reaction curve of a subject blood specimen in which presence or absence of a substance having coagulation factor VIII-substituting activity is required to be detected, where $i$ denotes the number of measurement points or time; and
   2) the following a) or b):

   a) determining $p_k$, which is a minimum of a point at which $V(i)$ reaches $X_k$ or higher when $V(i)$ reaches a maximum Vmax or therebefore, and $q_k$, which is a maximum of a point at which $V(i)$ reaches $X_k$ or higher when $V(i)$ reaches the maximum Vmax or thereafter, where $k$ represents a series of integers from 1 to **n,** n is an integer greater than or equal to two, $X_k$ is Vmax × $S_k$%, and $0 < S_k \leq 100$;
   b) determining a relativized maximum of $V(i)$.

2. The method according to claim 1, wherein the subject blood specimen is a blood specimen with no prolonged activated partial thromboplastin time (APTT).

3. The method according to claim 1, wherein the n is an integer from 5 to 50.

4. The method according to claim 1, further comprising calculating a parameter for analyzing the blood coagulation ability of the subject blood specimen, based on the $p_k$ or $q_k$.

5. The method according to claim 4,

   wherein the parameter is at least one selected from the group consisting of a pre-post average difference, CV, qp ratio, $vB(S_k)$, kurtosis $vAB(S_k)$, rate over time $vTB(S_k)$, $vAB(S_k)$ × VmaxT, $vAB(S_k)$ × APTT, parameters about relative values of $p_k$ and $q_k$, and their standard deviation indices (SDI), where
   the pre-post average difference represents (average of $q_a$ to $q_b$ - average of $p_a$ to $p_b$) / (average of $p_a$ to $p_b$ and $q_a$ to $q_b$) (where a and b are integers selected from the group consisting of 1 to n, n is as defined above, and a < b),
   the CV represents a coefficient of variation of $p_a$ to $p_b$ and $q_a$ to $q_b$ (where a and b are integers selected from the group consisting of 1 to n, n is as defined above, and a < b),
   the qp ratio represents $q_k/p_k$,

the vB($S_k$) represents an interval from $p_k$ to $q_k$,
the VmaxT satisfies V(VmaxT) = Vmax,
the APTT is an activated partial thromboplastin time (APTT) of the subject blood specimen,
the vAB($S_k$) = vH($S_k$)/vB($S_k$), and
the vTB($S_k$) = vB($S_k$)/VmaxT,
where
the vH($S_k$) is of the following equation,

$$vH(Sk) = \frac{\sum_{i=pk}^{qk}\left(i \times V(i)\right)}{\sum_{i=pk}^{qk} i}$$

6. The method according to claim 5,

   wherein the parameters about the relative values of the $p_k$ and $q_k$ are slopes of linear regression lines of a plot from $p_a$ to $p_b$ of the subject blood specimen and a plot from $q_c$ to $q_d$ of the subject blood specimen with respect to $p_a$ to $p_b$ of a reference specimen and $q_c$ to $q_d$ of a reference specimen, respectively (where a, b, c, and d are integers each independently selected from the group consisting of 1 to n, a < b, c < d, and n is as defined above), and
   the reference specimen is a blood specimen to which no drug having FVIII-substituting activity has been administered.

7. The method according to claim 1, further comprising calculating a parameter for analyzing the blood coagulation ability of the subject blood specimen, based on the relativized maximum of V(i).

8. The method according to claim 7,

   wherein the parameter is at least one selected from the group consisting of corrected Vmax, corrected Vmax $\times$ VmaxT, corrected Vmax $\times$ APTT, and their standard deviation indices (SDI),
   the corrected Vmax is the relativized maximum of V(i),
   the VmaxT satisfies V(VmaxT) = Vmax, and
   the APTT is an activated partial thromboplastin time (APTT) of the subject blood specimen.

9. The method according to any one of claims 4 to 8, further comprising detecting the presence or absence of the substance having coagulation factor VIII-substituting activity in the subject blood specimen, based on the parameter.

10. The method according to claim 9, further comprising outputting a result of the detecting the presence or absence of the substance having coagulation factor VIII-substituting activity.

11. The method according to claim 1, further comprising:

    measuring the activated partial thromboplastin time (APTT) of a blood specimen; and
    selecting the blood specimen with no prolonged APTT, as the subject blood specimen in which the presence or absence of the substance having coagulation factor VIII-substituting activity is required to be detected.

12. The method according to claim 1, wherein the substance having coagulation factor VIII-substituting activity is a bispecific antibody substituting for a cofactor function of coagulation factor VIII.

13. The method according to claim 1, wherein the substance having coagulation factor VIII-substituting activity is emicizumab.

14. A program for implementing the method according to claim 1.

15. A device for implementing the method according to claim 1.

16. A system for implementing the method according to claim 1, the system comprising: a program for implementing the method according to claim **1;** and a device controlled by the program.

17. The device according to claim 15 or the system according to claim 16, further comprising an output unit for outputting a result of analysis of the blood coagulation ability of the blood specimen by the method according to claim 1.

## Fig. 1

## Fig. 2

## Fig. 3A

```
ACQUIRE R(i)                                    S1

CALCULATE APTT FROM R(i).                       S2

COMPARE                                         S3
APTT WITH UPPER LIMIT OF
REFERENCE RANGE                  APTT > UPPER LIMIT

APTT ≤ UPPER LIMIT

CALCULATE V(i) FROM R(i)                        S4

CALCULATE ONE OR MORE                           S5
PARAMETERS FROM V(i)

COMPARE                                         S6
PARAMETER(S) WITH
THRESHOLD               NONE SATISFY THRESHOLD

AT LEAST ONE SATISFIES THRESHOLD

OUTPUT APTT CALCULATION RESULT TO    S7    NORMAL OUTPUT OF APTT      S8
WHICH FVIII-SUBSTITUTING SUBSTANCE          CALCULATION RESULT
DETECTION INFORMATION IS ADDED
```

EP 4 644 905 A1

Fig. 3B

24

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

## Fig. 18

Fig. 19

Fig. 20

## INTERNATIONAL SEARCH REPORT

| International application No. |
| --- |
| **PCT/JP2023/047187** |

### A. CLASSIFICATION OF SUBJECT MATTER

*G01N 33/86*(2006.01)i
FI: G01N33/86

According to International Patent Classification (IPC) or to both national classification and IPC

### B. FIELDS SEARCHED

Minimum documentation searched (classification system followed by classification symbols)

G01N33/86

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| X | WO 2022/054819 A1 (SEKISUI MEDICAL CO., LTD.) 17 March 2022 (2022-03-17) see entire text, all drawings, in particular, claims, paragraphs [0008], [0019], [0020], [0029]-[0044], [0066]-[0075] | 1-6, 9-17 |
| A | | 7-8 |
| A | 野上 忠嗣, 特集: 血友病診療・最近の話題 血友病A患者へのエミシズマブ使用時の止血モニタリング, 日本血栓止血学会誌, 2021, vol. 32, no. 1, pp. 3-11, https://doi.org/10.2491/jjsth.32.3 (NOGAMI, Keiji. Hemostatic monitoring at the use of Emicizumab for hemophilia A. Japanese Journal of Thrombosis and Hemostasis.) see entire text, in particular, p. 5, left column, 4. Emicizumab 定期投与下における凝固系検査への影響 etc. non-official translation (Impact of coagulating system test in periodic Emicizumab dosage) | 1-17 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **12 March 2024** | **26 March 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**

International application No.

**PCT/JP2023/047187**

## C. DOCUMENTS CONSIDERED TO BE RELEVANT

| Category* | Citation of document, with indication, where appropriate, of the relevant passages | Relevant to claim No. |
|---|---|---|
| A | 野上恵嗣, 【第60回日本小児血液・がん学会学術集会】シンポジウム5: 血友病の現状と今後の治療選択 血友病インヒビター-A患者の治療戦略, 日本小児血液・がん学会雑誌, 2019, vol. 56, no. 3, pp. 282-286 (NOGAMI, Keiji. 60th Meeting of the Japanese Society of Pediatric Hematology/Oncology, Symposium 5: Recent treatment strategy for hemophilia A patients who developed inhibitor. Japanese Journal of Pediatric Hematology/Oncology.) see entire text, etc. | 1-17 |
| A | ランチョンセミナー3エミシズマブ投与下における止血モニタリングの現状と課題. 日本血栓止血学会誌, vol. 31, no. 2, p. 192 non-official translation (Lunch seminar 3. Current status and challenges of hemostasis monitoring in Emicizumab dosage. Japanese Journal of Thrombosis and Hemostatis.) see entire text | 1-17 |
| P, X | 前田佑華, 他6名, APTT凝固波形解析によるEmicizumab含有検体の鑑別法の開発, 日本検査血液学会雑誌, 2023, vol. 24, S154, O18-1 non-official translation (MAEDA, Yuka et al. Using APTT clot waveform analysis in development of identification methods of samples containing Emicizumab. Journal of the Japanese Society for Laboratory Hematology.) see entire text, etc. | 1-17 |

Form PCT/ISA/210 (second sheet) (July 2022)

**INTERNATIONAL SEARCH REPORT**
Information on patent family members

International application No.

**PCT/JP2023/047187**

| Patent document cited in search report | Publication date (day/month/year) | Patent family member(s) | Publication date (day/month/year) |
|---|---|---|---|
| WO 2022/054819 A1 | 17 March 2022 | EP 4212880 A1 <br> see entire text, all drawings, in particular, claims, paragraphs [0008], [0019], [0020], [0033]- [0049], [0091]-[0101] <br> CN 116057383 A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014050926 A
- WO 2016170944 A
- JP 2017106925 A
- JP H6249855 A
- WO 2021132552 A
- WO 2021177452 A
- WO 2021206107 A